# EUROPEAN PATENT SPECIFICATION

(11) **EP 1 871 900 B1**
(45) Date of publication and mention of the grant of the patent: **10.02.2010**
(21) Application number: 06724120.8
(22) Date of filing: 07.04.2006
(51) Int. Cl.: C12Q 1/68

(54) **USE OF NOVEL HNF4ALPHA TARGET GENES AND THEIR GENE PRODUCTS**
VERWENDUNG NEUER HNF4ALPHA-ZIELGENE UND IHRER GENPRODUKTE
UTILISATION DES NOUVEAUX GENES CIBLES DE HNF4ALPHA ET LEUR PRODUITS GENIQUES

(30) Priority: 14.04.2005 EP 05090108
(43) Date of publication of application: 02.01.2008
(73) Proprietor: Fraunhofer-Gesellschaft zur Förderung der angewandten Forschung e.V., 80686 München (DE)
(72) Inventor: NIEHOF, Monika, 30161 Hannover (DE); BORLAK, Jürgen, 31275 Lehrte/OT Immensen (DE)
(74) Representative: Baumbach, Friedrich
(86) International application number: PCT/EP2006/003179
(87) International publication number: WO 2006/125493

(56) References cited:
- WO-A-98/23780
- WO-A-03/016559
- ODOM DUNCAN T ET AL: "Control of pancreas and liver gene expression by HNF transcription factors." SCIENCE (WASHINGTON D C), vol. 303, no. 5662, 27 February 2004 (2004-02-27), pages 1378-1381, XP002370770 ISSN: 0036-8075 cited in the application
- NIEHOF M ET AL: "RSK4 AND PAK5 ARE NOVEL CANDIDATE GENES IN DIABETIC RAT KIDNEY AND BRAIN" MOLECULAR PHARMACOLOGY, BALTIMORE, MD, US, vol. 67, no. 3, March 2005 (2005-03), pages 604-611, XP009047498 ISSN: 0026-895X cited in the application
- WANG H ET AL: "Hepatocyte nuclear factor 4alpha regulates the expression of pancreatic beta -cell genes implicated in glucose metabolism and nutrient-induced insulin secretion." THE JOURNAL OF BIOLOGICAL CHEMISTRY. 17 NOV 2000, vol. 275, no. 46, 17 November 2000 (2000-11-17), pages 35953-35959, XP002370771 ISSN: 0021-9258
- NAIKI TAKAFUMI ET AL: "Analysis of gene expression profile induced by hepatocyte nuclear factor 4alpha in hepatoma cells using an oligonucleotide microarray" JOURNAL OF BIOLOGICAL CHEMISTRY, vol. 277, no. 16, 19 April 2002 (2002-04-19), pages 14011-14019, XP002370772 ISSN: 0021-9258 cited in the application
- SILANDER K ET AL: "GENETIC VARIATION NEAR THE HEPATOCYTE NUCLEAR FACTOR-4ALPHA GENE PREDICTS SUSCEPTIBILITY TO TYPE 2 DIABETES" DIABETES, NEW YORK, NY, US, vol. 53, no. 4, April 2004 (2004-04), pages 1141-1149, XP001205725 ISSN: 0012-1797
- ISAACSON KERKVLIET N.; KIMELDORF DONALD J.: 'Inhibition of Tumor Growth in Rats by Feeding a Polychlorinated Biphenyl, Aroclor 1254' BULLETIN OF ENVIRONMENTAL CONTAMINATION AND TOXICOLOGY vol. 18, no. 2, August 1977, NEW YORK, pages 243 - 246

## Description

The invention relates to the use of novel target genes of the transcription factor HNF4α and their gene products or their functions to screen for and to identify drugs directed against metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth. Furthermore the invention concerns methods to use thereof and substances which regulate said gene products.

### Background of the invention

Certain metabolic diseases, like for example diabetes type II, are widespread diseases. Prognoses predict an amount of 366 million of diabetic patients worldwide (4.4 % of the population) in 2030. This is twice as much patients as in 2000. Additionally to a defective regulation of the glucose metabolism diabetic patients suffer from diabetic nephropathy, diabetic neuropathy, diseases of the retina, cardiovascular diseases and an impairment of hearing. Worldwide great efforts are made to understand the molecular basis of diabetes. There is evidence that a non-functional HNF4α gene plays a critical role for the development of diabetes. The zinc-finger transcription factor HNF4α is of pivotal importance for liver development and hepatocellular differentiation and plays an essential role in a regulatory circuity to control a wide range of metabolic processes.

HNF4α is a zinc-finger transcription factor and a member of the hepatic transcription factor network. It is a key player in liver biology and drives hepatocyte differentiation (7;28;44;54). Specifically, HNF4α contacts regulatory elements of several genes of various metabolic pathways including carbohydrate, lipid, steroid, xenobiotic and amino acid metabolism, respectively (63;66). This factor also participates in the glucose-dependent insulin secretory pathways (63;66). The role of HNF4α in glucose metabolism is particularly obvious through its functional link to diabetes. Indeed, one form of a monogenetic disorder termed maturity onset diabetes of the young (MODY) was mapped to mutations within the HNF4α gene (MODY-1). There is conclusive evidence for a unique and pivotal role of HNF4α in pancreatic β-cell function (63;66) and HNF4α dysfunction is strongly associated with multifactorial Type 2 diabetes (45;65). Further, metabolic disposalof endogenous compounds including cholesterol and/or fatty acids relies on the proper function of cytochrome P450 (CYP) monooxygenases and the expression of a broad range of CYP-isozymes is regulated by HNF4α as well (33). Notably, treatment of rat hepatocyte cultures with Aroclor 1254, i.e. a complex mixture of polychlorinated biphenyls, resulted in the simultaneous induction of HNF4α and of several CYP isoforms and this points to a coordinate response in the regulation of HNF4α and of genes targeted by this factor (4). Besides its pivotal functions in liver metabolism, HNF4α also targets genes in other tissues and organs, such as kidney, intestine and colon (66). In general, HNF4α is a dominant regulator for an epithelial phenotype, triggers de novo formation of functional tight junctions and contributes to epithelial cell polarity (8). Because of its role in the differentiation of epithelium, it is probable that HNF4α plays an additional role in the control of cell proliferation. Indeed, Chiba et al (9) reported overexpression of HNF4α to inhibit cell growth in F9 cells presumably due to exaggregated expression of cyclin-dependent kinase inhibitor p21^{CIP1/WAF1}. As of today a total of 76 genes targeted by HNF4α have been studied in some detail. Results from these investigations suggest 95 bona fide recognition sites for HNF4α. Different experimental strategies were, however, employed to identify novel HNF4α gene targets and this included an in-silico approach (17) or transfection of HNF4α into a human hepatoma cell line (49) or in a rat insulinoma cell line (70). Most recently, the ChIP-chip assay enabled a comprehensive search for novel HNF4α candidate genes (53) and this approach yielded an unexpected high number of putative candidate genes, i.e. 1575. Though, there is concern about proper validation of results obtained by the ChIP-chip assay, it unprecedently demonstrates the tremendous versatility of HNF4α in contacting promoters of many different genes. The invention is related on the successful search and surprising identification of novel HNF4α gene targets by the use of the chromatin immunoprecipitation (ChIP) assay and cloning of targeted DNA.

The aim of the present invention is therefore to make available the fast and efficient screening and identifying of drugs directed against metabolic diseases, including type 1 and/or type 2 diabetes mellitus, and/or late stage complications of diabetes-caused diseases, including diabetic nephropathy, hearing dysfunction, diabetic neuropathy, cardiovascular diseases or diseases of the retina, and/or tumor growth as well as beneficial methods to use thereof and substances for treating said diseases. To this end, the implementation of the actions as described in the claims provides appropriate means to fulfill these demands in a satisfying manner.

Dysfunction of HNF4α may lead to disease and an identification of genes targeted by this factor provides insights into mechanisms of disease. In accordance with the invention thirteen new HNF4α target genes were found (C20orf13, KIAA0774, EPS15R, PLCB1, UGTREL1, RSK4, PAK5, FMR2, NEB, NFYC, KCNQ4, PRPF3, TRPC1). These genes were identified by means of molecular biological and molecular genetic methods. The genes code for various biological functions (metabolism, regulation of cell cycle and signal transduction, differentiation, ion channels, mRNA processing, see table A) and are thus important for the therapy of metabolic disorders, diabetic diseases and tumor growth.
The HNF4α target genes RSK4 and PAK5 are already reported. These genes encode kinases which are selective regulated in animal models of diabetes. The kinases RSK4 and PAK5 are of fundamental importance for the therapy of diabetic nephropathy and neuroropathy (Niehof, M. and Borlak, J. 2005, RSK4 and PAK5 are novel candidate genes in diabetic rat kidney and brain. Mol. Pharmacol. 67 604-611.; European Patent application 04016948.4).
In the present invention, additionally eleven new HNF4α target genes are described.
The gene TPRC1 encodes a non-selective cation channel, which is activated via a receptor or after emptying of intracellular stores. The TPRC1-channel controls the influx of calcium into the cell. TPRC1 proteins are expressed in most cells and are the most important channels for the uptake of calcium into the cell. TPRC1 is for example expressed in the pancreas and the kidney. In the pancreas TPRC1 can support the calciumhomeostasis. A defective regulation of TPRC1 can effect the secretion of insulin.
It was shown that HNF4α and TPRC1 are regulated in animal models of diabetes. Thus, TPRC1 is a candidate gene for the treatment of diabetic nephropathy.
KCNQ4 encodes a voltage-dependent potassium channel. This channel is expressed in sensory haircells of the cochlea. The dysfuction of this channel can cause loss of hearing. Impairments in hearing are one of the symptoms caused by diabetes.

The HNF4α transcription factor targets the genes EPS15R and PLCB1, which are involved in the regulation of signal cascades. Via these genes HNF4α controls the cell cycle. It was found that the EPS15R protein binds to the EGF-receptor. This knowledge can be used to develop strategies to treat tumors.

### Summary of the invention

The invention is realized according to the claims.
With the help of molecular biological and molecular genetic methods new HNF4α target genes (C20orf13, KIAA0774, EPS15R, PLCB1, UGTREL1, RSK4, PAK5, FMR2, NEB, NFYC, KCNQ4, PRPF3, TRPC1) were identified. These 13 genes encode for gene products, herewith identified as being important for cell cycle regulation, signal transduction, metabolism, nutrient transport, ion channels, development and mRNA splicing. An overview of these genes is given in Table A, providing also the links to their database entries and sequences which herewith are referred to this invention.

**Table A. Data base entries and biological relevance of the new HNF4α target genes and their gene products.**

| gene name | Swiss-Prot/mRNA NCBI | molecular function | biological process |
|---|---|---|---|
| C20orf13 | Q9H6P5/NM_017714 | asparaginase activity (prediction) | metabolism / glycoprotein catabolism (prediction) |
| KIAA0774 | 094872/XM_166270 | hypothetical protein | Unknown |
| EPS15R (EPS15L1) | Q9UBC2/NM_021235 | receptor activity | Endocytosis signal transduction |
| PLCB1 | Q9NQ66/NM_015192 | phospholipase activity | cell communication / signal transduction regulation of cell cycle |
| UGTREL1 (SLC35B1) | P78383/NM_005827 | UDP-galactose transporter activity | Transport |
| RSK4 (RPS6KA6) | Q9UK32/NM_0144496 | protein kinase activity | cell communication I signal transduction |
| PAK5 (PAK7) | Q9P286/NM_045653 | protein kinase activity | cell communication / signal transduction |
| FMR2 | P51816/NM_002025 | transcription regulator activity (prediction) | Development |
| NEB | P20929/NM_004543 | structural constituent of muscle | Development |
| NFYC | Q13952/NM_014223 | transcription regulator activity | transcription redox response |
| KCNQ4 | P56696/NM_004700 | ion channel activity | ion transport / ion channel |
| PRPF3 (HPRP3, PRP3) | 043395/NM_004698 | mRNA splicing factor activity | nuclear mRNA splicing |
| TRPC1 | P48995/NM_003304 | ion channel activity | ion transport I ion channel |

The identification of these genes and their fundamental importance for a therapy of metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth is described. Agents can now be identified for the control of genes,where regulation is for example disturbed by these diseases. Furthermore, it enables the screening and identification of drugs controlling mutants or variations of these genes or partial sequences playing a role in the pathologic outcome of metabolic dysfunctions.

Therefore new therapeutic concepts are possible for the treatment of metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth.
The term gene in this invention comprises single or double stranded DNA, possibly combined with regulatory elements for their transcription, including sequences as being reffered by the data base codes (s. Table A), but also the use of accordant single stranded (sense or anti-sense) DNA is possible. The term mutant in this context is related to nucleic acid sequences including at least one genetic mutation, for example sequence or chromosomal mutations, compared to the native sequence. The term variation is directed towards all possible parts and/or their combinations of the referred sequences, as well as variations leading to processing or splicing variants or to combinations of the referred mRNA or to respective mutants, as well as sequences including naturally or unnaturally labelled nucleotides, for example methylation patterns. The term part thereof concerns shorter sequences related from the referred sequences, for example coding for a protein domain, but also can include for example parts of introns and/or exons or their combinations, as well as primers for the use in a polymerase chain reaction, and also the use of sequences interacting with mRNA or siRNA is practical, but also interaction with non natural molecules, for example PNA sequences, is appropriate. All of the mentioned elements in the invention or their combinations can also be used appropriately in a non natural environment, for example as being part of a plasmid / transcription vector and/or being expressed in cell culture and/or in an in vitro translation system. Furthermore, said sequences can also be used in combinatorial screening systems, for example in yeast-two-hybrid, phage display, enzyme-function-reassembly, random mutagenesis or different systems, as well as being used by array techniques, for example in micro arrays or spot syntheses systems.

An overview of the function of the new HNF4α regulated gene products is given in Table A. Therein, furthermore, an outline of the biological processes these proteins are involved in, and data base entries is supplied and referred herewith in this invention.
The present invention is now adding the knowledge that these gene products are involved in the complex regulation of certain metabolic processes, which, if dysregulated, can cause metabolic diseases, particularly type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth. Hence, these gene products or, for example their mutants, are now usable as targets for the identification of pharmaceutical leads concerning said diseases. Also, their variations, for example the gene products being phosphorylated or glycosylated, or parts thereof is approriate to derive means, and in particular molecules, for example peptides or peptides including non natural building blocks or D-amino acids and/or peptidomimetics having the desired properties.
Therefore, the inventive use of said gene products enables an easy and effective screening and identification of agents for the treatment of metabolic diseases.
Furthermore, as their dysregulation can lead to metabolic diseases, the invention is also related to mutants, variations and parts of these gene products.

In general, the inventive use of the identified HNF4α target genes and/or their gene products as well as their related/derived molecules furthermore allows for the first time easy and straightforward strategies for identifying means for ameliorating metabolic disorders / dysfunctions, particularly said diseases, if combinations of several genes or gene products or their derived molecules / sequences are used.
The term gene products comprises all possible translational products being coded by the above specified nucleotide sequences their mutants. Moreover, it relates to all possible unfolded and folded proteins, protein complexes or protein derived structures, comprising glycoproteins or lipoproteins, including amino acid sequences which are coded by said nucleotide sequences. The term variations concerns all possible posttranslational modifications, for example phosphorylations or the formation of disulfide bridges. The term mutant in this context relates to all sequences differing from the native sequence by at least one amino acid residue, or any other modifications leading to a misfolded or dysfunctional structure. The terms parts thereof also includes fragments or enzymatic cleavage products.
In other aspects of the invention, the goal oriented use of genes and/or their gene products being coded by the genes TRPC1, KCNQ4, EPS15R, PLCB1, C20orf13 or UGTREL, and/or mutants and/or variations and/or parts thereof for the search and identification of drugs being directed against the specified diseases is described.

In one aspect of the invention one gene and/or gene products being coded by the gene and/or its mutations and/or variations and or parts thereof are selected and used for the screening and identifying of drugs concerning a certain application area, according to the claims 3-8. This aspect has the advantageous characteristic that it enables a simple straightforward screening and identification procedure.

In another aspect of the invention one gene and/or gene products being coded by the gene and/or its mutations and/or variations and or parts thereof are selected and used for the screening and identifying of drugs by termining the function of said gene and/or gene products in certain fields, according to the claims 9-20. This aspect has the preferable characteristic that it eases the identification procedure by reducing the parameters for the screening procedure.
The functions said genes or their gene products are involved in and which are appropriate for the screening and identification of said drugs are summarized as follows:

### EPS15R function

- in binding to EGF-receptor
- in endocytosis and proteosomal degradation of EGF-receptor
- in inhibition of EGF-receptor
- in EGF-receptor mediated signal transduction
- in regulation of tumor growth

### PLCB1 function

- in activation through G-proteins
- in stimulation of PKC
- in nuclear activation through ERK1 and ERK2
- in regulation of differentiation
- in regulation of proliferation
- in regulation of cell cycle
- in regulation of tumor growth

### C20orf13 function

- in taspase function
- in catalysing glycoprotein metabolism
- in decrease of deamidation of asparagine
- in regulation of tumor growth
- in cellular differentiation
- in regulation of HOX-genes
- in organogenesis / development

### UGTREL1 function

- in glycoconjugate synthesis
- in effects on cell adhesion and tumor growth

### RSK4 function

- in mediating MAP/ERK signal transduction
- in regulation of gene expression by phosphorylation of transcription factors (namely CREB, CBP/p300, ERα, IKBα/NFkB, c-Fos)
- in regulation of cell cycle
- in regulation of cell proliferation
- in regulation of cell differentiation
- in the treatment of nephro- and neuropathies

### PAK5 function

- in mediating effects of rho-proteins (such as cdc42 or p21-rac1)
- in regulation of MAPK signaling pathways
- in regulation of cytoskeletal dynamics
- in regulation of cell cycle
- in regulation of cell proliferation
- in regulation of cell differentiation
- in the treatment of nephro- and neuropathies

### FMR2 function

- in transcriptional activation of genes
- in regulation of cell differentiation of for instance neuronal cells

### NEB function

- in maintaining structural integrity of cardiac and skeletal muscle

### NFYC function

- in transcriptional activation of genes
- in regulation through cellular redox potential
- in regulation based on redox response

### KCNQ4 function

- in hearing impairment and hearing lost including late stage complications of metabolic diseases
- in generating potassium currents and membrane potentials in brain to transduce signals
- in generating faultless potassium currents and membrane potentials in other organs, e.g. heart to transduce signals

### PRPF3 function

- in participating in pre-mRNA splicing as component of the spliceosome
- as part of faultless gene expression
- in treatment of disorders linked to RNA-spliceosome

### TRPC1 function

- as part of heterodimers with other TRP proteins
- in primary mode of Ca²⁺ entry after receptor activation or after store-dependent activation
- in calcium homeostasis including kidney and pancreas
- as non-selective cation channels in beta-cells
- in insulin secretion by regulating pancreatic beta-cell plasma membrane potential in a K_{ATP} channel independent manner
- in glucose-signalling
- in the treatment of metabolic disorders including diabetes and nephropathy

In yet another aspect of the invention drugs regulating the expression of one or more genes and/or the function of one or more gene products or molecules derived thereof are used for the treatment or for the production of means for the treatment of metabolic diseases, according to the claims 21-23. This aspect has the favourable aspect that it allows a fast and easy way to realise the drugs identified towards a product that can be sold.

A further aspect of the invention concerns the use of said genes and/or related molecules and/or gene products or related molecules for preparing a medication for the treatment of metabolic diseases, according to claim 24. This aspect has the preferable characteristic that it allows an easy application of said identified drugs, for example in a beneficial dose.

Yet a further aspect of the invention relates to a method for the identification of compounds directed against metabolic diseases wherein genes and/or their related molecules and/or their gene products and/or derived structures are used as targets and changes in the expression and/or function are determined, according to claim 25. This aspect has the favourable characteristic that it enables a fast and efficient identification procedure and further reduces the screening expense.

Another aspect of the invention is related to methods for the treatment of metabolic diseases, according to the claims 26 and 27. This aspect has the advantageous aspect that it allows the treatment of said diseases by the use of compounds directly directed against the chosen targets, for example antibodies or derived structures.

Yet another aspect of the invention concerns an method for the treatment of metabolic diseases by the use of agonistic compounds according to the claims 28-32. This aspect has the preferable characteristic that it allows the simple treatment of said diseases, for example by the use of shortened sequences or derived molecules, which can compensate or enhance a natural function.

A further aspect of the invention relates to a method for the treatment of metabolic diseases by the use of blocking / antagonistic substances according to the claims 33-36. This aspect of the invention has the favourable characteristic that an upregulated dysfunction can be easily reduced.

Yet another aspect of the invention features a method for the treatment of metabolic diseases by reducing the overexpression of a normal gene, according to the claims 35 and 36. This aspect has the advantageous characteristic that the natural function of a dysregulated gene can be easily downregulated or restored.

Another aspect of the invention provides substances regulating said genes or gene products, according to the claims 37 and 38. This aspect has the preferable characteristic that it enables the easy storage, preparation or use of compounds being able to ameliorate metabolic diseases

Other features and advantages will be apparent from the following detailed description.

### Detailed disclosure of the invention

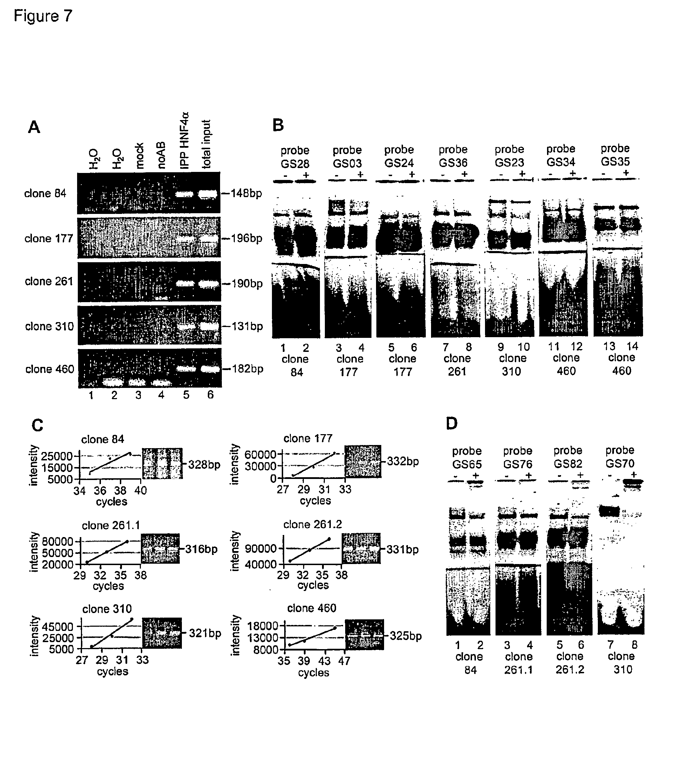

Several independent investigations provide evidence for Caco-2 cells to be valuable for functional studies on HNF4α (27;29). The invention relates on 13 novel genes targeted by HNF4α and was requested in three independent ChIP experiments by repetitive identification of novel gene targets. Additionally, and whenever possible, *in* vitro binding of HNF4α to recognition sites of candidate genes was confirmed by EMSA and mRNA expression of gene targets was verified by quantitative RT-PCR.
Notably, the cDNA of C20orf13 (12) was sequenced from colon mucosa and HepG2 cells and codes for a protein with predicted asparaginase activity, which might participate in glycoprotein metabolism. Likewise, UGTREL1 (31) codes for an isoform of the UDP-galactose transporters and carries nucleotide sugars into the Golgi apparatus to enable glycoconjugate synthesis. Thus, identification of C20orf13 and UGTREL1 provide further evidence for HNF4α to function as a master regulator in metabolism (63;66). A further gene targeted by HNF4α is an isoform of phospholipase C (PLCB1). This protein is involved in cell communication and signal transduction and belongs to one of several phospholipase C-beta isoforms (5). Specifically, PLCBs catalyze the hydrolysis of phosphatidylinositol-bisphosphate (PIP₂) to generate the second messengers diacylglycerol (DAG) and inositol-triphosphate (IP₃) with subsequent Ca²⁺ mobilization and proteinkinase C (PKC) activation. It therefore facilitates cell proliferation and differentiation (5). Within the plasma membrane PLCB1 is activated by members of the alpha-q family of G-proteins (5). However, phospoinositide-metabolism also occurs in the nucleus and nuclear PLCB1 is a physiological target of ERK1 and ERK2 (18). PLCB1 is basically expressed in all human tissues tested so far and nuclear PLCB1-signaling based on increased expression and activity was recently linked to myogenic differentiation (18). It is of considerable importance that the gene coding for an EPS15R (38), epidermal growth factor receptor substrate, was targeted by HNF4α. This protein plays a pivotal role in receptor down-regulation and in clathrin-mediated endocytosis as well as degradation of activated EGF-receptors (18). Thus, HNF4α targets partners of cell cycle regulation presumably with to aim cellular differentiation, rather than cell proliferation. HNF4α also targets KIAA0774. The cDNA of this gene (48) was sequenced from brain and encodes for a hypothetical protein with as yet uncertain function, when assessed on the basis of homology. Furthermore, two kinases were identified, i.e. RSK4 and PAK5, which regulate, in part, cell cycle and signal transduction (51). Because of their importance in diabetic neuro- and nephropathy these kinases were studied in detail in an streptozotocin disease model and reported comprehensively elsewhere (51). Taken collectively, this reports evidence for HNF4α to target genes with novel functions, which are beyond its master functions in metabolism and nutrient transport.
As discussed above, *in vitro* binding of HNF4α to candidate genes (Table 6) was confirmed by EMSA, but for some targets (FMR2, NEB, NFYC, KCNQ4, PRPF3, TRPC1, see Table 7) *in vitro* binding to the ChIP confirmed fragments could not be demonstrated. Nonetheless, these genes are faithful HNF4α targets, because they have been verified in separate immunoprecipitation experiments (Fig. 7A). Indeed, during initiation of transcription, a specific transcription factor interacts with several coactivators and basal factors. Binding of different transcription factors as part of a multiprotein complex leads to combinatorial control of gene expression. As formaldehyde crosslinks lead to both, protein-DNA and protein-protein fixation, ChIP-assays allow for the study of a three-dimensional, higher order structure. Thus, immunoprecipitated HNF4α might not necessarily contact DNA directly, but a cooperating partner, which was in contact through multimeric protein interactions with chromatin. Indeed, Hatzis and Talianidis (27) used the ChIP-assay to investigate the order of recruitment of transcription factors to HNF4α enhancer and promoter and found the promoter to contact long distance (6 kb) immunoprecipitated enhancer associated transcription factors. Thus, the CHIP assay allowed for protein-protein interactions of factors contacting enhancer and promoter regulatory regions. Consequently, the genes listed in Table 7 were fished by crosslinked protein-protein complexes.
Indeed, Fragile X mental retardation protein 2 (FMR2) is shown to be targeted by HNF4α and this protein is a member of a new family of putative transcription activators. Defects in FMR2 are the molecular cause of FRAXE mental retardation (25) and expression of lacZ-constructs in knock-out animals provide evidence for FMR2 to be expressed in several tissues with as yet unknown functions (25). Further, Nebulin (14) is also regulated by HNF4α and codes for a giant muscle protein with functions in actin cytoskeleton. Its expression in tissues other than muscle is, however, unknown. Additionally, HNF4α targets PRPF3, which codes for a component of the spliceosome and participates in pre-mRNA splicing (6). It is highly expressed in liver, kidney, blood and retina and mutations in PRPF3 are implicated in autosomal retinitis pigmentosa (6). TRPC1, transient receptor potential cation channel, subfamily C, member **1**, codes for a nonselective cation channel, is widely expressed and allows plasma membrane calcium influx to occur in response to intracellular depletion or to activation by G(q)-coupled receptors (10). Specifically, nonselective cation channels play a role in insulin secretion and regulate pancreatic beta-cell plasma membrane potential, Ca(2+) homeostasis and thus glucose signaling (58). Qian et al (58) proposed TRPCs as good candidates for, as yet, not well-characterized nonselective cation channels in beta-cells. HNF4α regulates several genes involved in glucose metabolism (63) and participates in the glucose-dependent insulin secretory pathways (63;66). HNF4α dysfunction does, however, lead to multifactorial Type 2 diabetes (45;65) and TRPC1 might be an important disease associated HNF4α candidate gene target during diabetes. Furthermore, it was verified by immunoprecipitation of a region located around 7.5 kb upstream of potassium voltage-gated channel, KQT-like subfamily, member 4 (KCNQ4) and around 4.7 kb downstream of nuclear factor Y (NFYC). KCNQ4 belongs to the potassium channel family and regulates the excitability of sensory cells of the cochlea. Defects in KCNQ4 are a cause of nonsyndromic sensorineural deafness type 2, an autosomal dominant form of progressive hearing loss (73). It is expressed in the outer sensory hair cells of the cochlea and also slightly in heart, brain and skeletal muscle with as yet unknown functions. Hearing impairment has been reported to be one of the late complications of diabetes. It appears to be a multifunctional process with cochlear and nerve involvement (15). Therefore, KCNQ4 might represent a further novel HNF4α gene target deregulated during diabetes, but requires further studies. NFYC is one subunit of the highly conserved transcription factor NFY, which binds as trimeric complex with high specificity to CCAAT boxes in the promoter regions of a variety of genes (61). During hypoxia HNF4α physically interacts with HIF-1α, which results in increased erythropoietin gene expression (72), and furthermore, HNF4α regulates redox-mediated iNOS (inducible NO synthase) expression (26). Interestingly, transcriptional activity of NFY is regulated by the cellular redox potential (47) and this might implicate an additional link of HNF4α to redox response. The findings given in Table 7 are likely to be the result of immunoprecipitated protein-protein crosslinks (see above). In addition, it was tested only whether predicted recognition sites of HNF4α in the promoter of these genes can be confirmed, but did not investigate their in vivo binding. Indeed, in vitro binding to putative promoter sites of FMR2, KCNQ4, NFYC, and PRPF3 could be demonstrated. The results therefore provide strong evidence for these genes to be regulated by HNF4α.

HNF4α contacts DNA exclusively as a homodimer and physically interacts with members of the basal transcription machinery, cofactors and several transcription factors (63;66). Protein-protein interaction of HNF4α homodimers with other transcription factors is well documented for HNF1α (41) and COUP-TF (40). Because of these interactions it was analyzed *in vitro* binding of COUP-TF and HNF1α to the newly identified HNF4α binding sites. Besides HNF4α, COUP-TF has the potential to contact HNF4α recognition sites and may compete with HNF4α for the same sites (63;66). Neither COUP-TF nor HNF1α was bound to the novel HNF4α target sites as determined by EMSA and super shift assays. In addition, it was used the Transfac matrixes for COUP-TF and HNF1α with a cut-off matrix similarity of 0.78 to minimize false positive matches (min FP). Applying these matrixes was not suggestive for COUP-TF or HNF1α binding to immunoprecipitated fragments. The results therefore provide strong evidence for specificity of HNF4α binding.
Indeed, HNF4α acts in concert with other, mainly liver specific transcription factors (63) and the described analysis for binding sites of other transcription factors in proximal promoters with Transfac matrixes setting of minFP cut off resulted in promising, but experimentally unproven binding sites. Based on this computation, binding sites for HNF1, HNF3, C/EBPα, C/EBPβ and GATA-4 surrounding the HNF4α sites in proximal promoters (-1 to -3500bp) of the newly identified gene targets are predicted.

In fact, ChIP cloning in combination with carefully performed target confirmation is a time consuming process, but allows for thorough validation of novel gene targets. However, there are methodological considerations that need to be addressed. Indeed, until now ChIP cloning of formaldehyde crosslinked nucleoprotein complexes was scarcely employed. ChIP cloning was, however, used to study E2F (9 targets) (74), E2A (8 targets) (23), Egr1 (1 target) (11), EWS/ATF-1 (6 targets) (32), RUNX1 (1 target) (30), BARX2 (21 targets) (69), Smad4 (1 target) (64), STAT5a/b (9 targets) (50) and RUNX2 (4 targets) (2). All gene candidates were confirmed by independent ChIP experiments and subsequent PCR analyses using clone-specific primers. Due to the fact that it is very difficult to completely avoid nonspecific immunoprecipitated DNA, validation of findings is extremely important (74). Some investigators reported similar or modified approaches to isolate genomic DNA, but did not apply independent ChIP experiments to confirm *in vivo* binding (37;56;60;62). This limits the value of findings as no proof of *in vivo* binding to immunoprecipitated DNA is presented. In strong contrast numerous independent experiments and thoroughly validated HNF4α gene targets were employed. Specifically, three independent ChIP experiments to annotate targets were requested. Prior to cloning it was further searched for known targets in immunoprecipitated DNA to proof specificity. The number of clones identified is in line with findings reported for other transcription factors based on the ChIP-cloning procedure (see above). Specifically, none of these studies cloned already known targets due to low amount of immunoprecipitated DNA and when compared with the expected number of genes targeted by a transcription factor. A further point of consideration is the even distribution of gene targets amongst different chromosomes. This demonstrates the utility of ChIP-cloning procedure in identifying genome wide targets. Also being identified were several targets for HNF4α within a chromosome and half of the cloned fragments were annotated to regions of 5 to 50kb upstream or downstream of coding DNA, whereas the other half of the cloned fragments were sequences derived from intron 1 or intron 2. Notably, Weinmann et al (74) identified promoter regions of three novel E2F targets from ChIP-cloned fragments, but most investigators detected rarely proximal promoter binding sites from ChIP-derived clones. For instance, Jishage et al (32) described the cloning of six confirmed EWS/ATF-1 targets, whereby only one target contained a promoter binding site, whereas three were located far away from transcription initiation site. In the study of Martone et al (46) NFkB binding sites were investigated by the "ChIP on a chip" assay. Specifically, the authors revealed binding of NFkB proximal to 5' ends, but observed binding with high frequency at many other sites, including introns (in total 40% of the sites) as well as sites distal to 5' prime end. Therefore, the distribution of binding sites for NFkB for human chromosome 20 is strikingly similar to the results with HNF4α. Indeed, approximately 90% of the well-known HNF4α binding sites are located within 3000bp upstream of the predicted start site of transcription (TSS) and 60% are located within 500bp upstream of the TSS. However, binding of specific transcription factors is not restricted to proximal promoter regions alone. It is clear by now that a typical animal gene may contain several enhancers located in 5' and 3' regulatory regions over distances of 100kb, in addition to binding sites within introns (43). Intronic enhancers have become well known in recent years and are located predominantly in intron 1 and intron 2 (42). In the past, searches for transcription factor binding sites were focused on proximal promoter region even though binding-sites for HNF4α were also described for the 5' enhancer regions of OTC (52), ApoB (1), and CYP3A4 (71) and the 3' enhancer regions of EPO (21) and ApoAl (3). Likewise, HNF4α contacts intronic enhancers in the case of aldolase B (24), apolipoprotein B (1) and adenosine deaminase (16).
Specifically, proximal promoters (-1 to -3500bp) of HNF4α ChIP-clones with Swiss Prot entry for putative binding sites and designed primer pairs for cloned fragments and studied predicted promoter binding sites in independent ChIP experiments were analyzed. *In vivo* HNF4α binding to EPS15R, KIAA0774 and TRPC1 was ChIP-verified for recognition sequences in the first intron and *in vivo* binding to PLCB1, UGTREL1, C20orf13, FMR2, NEB and PRPF3 was verified for promoter binding sites. Furthermore, it was ChIP verified *in vivo* HNF4α binding to a region around 7.5 kb upstream of KCNQ4 and around 4.7 kb downstream of NFYC. Once again, *in vitro* binding of HNF4α to promoter recognition elements of these targets was confirmed as well (see **Fig. 7D** **and Table 7**)

Noteworthy, several approaches were reported for identification of HNF4α gene targets and this included a bioinformatic approach (17), HNF4α overexpression (49;70) and ChIP-assays combined with microarrays (53). Indeed, Ellrot et al (17) developed an algorithm based on the Markow chain optimization method to scan the human genome for HNF4α binding sites. 71% of the resulting sites were confirmed by *in vitro* binding assays. Surprisingly, in this report the genes were not specified and *in vitro* binding alone is insufficient evidence and disregards (a) binding based upon synergy with other factors and (b) ignores *in vivo* accessibility to high order chromatin structure. For example, it was analyzed *in vitro* binding of 43 computational predicted HNF4α binding sites based on the ChIP cloned and sequenced DNA. Notably, it was able to confirm 15 binding sites only, based on EMSA. Indeed, algorithms developed by Transfac and Genomatix are weight matrix-based. Several matrixes were applied for the detection of binding sites and carried out independent experiments to confirm both *in vivo* and *in vitro* binding. However, the applied algorithm produced conflicting results with one recognition site being detected by the Transfac matrix only whereas an other site was detected by the Genomatix matrix only. By applying several algorithm false positive and false negative results were obtained. Therefore experimental confirmation is a must for target site validation. As shown here and by other investigators for c-myc (19) and E2F (36) in-silico approaches will be improved when the modular organization of regulatory regions into promoter models is incorporated (20). The most basic forms of regulatory modules are composite elements consisting of pairs of functional transcription factor binding sites that act synergistically (35). These composite modules were successfully used for database searches that were independent of direct sequence similarity (39). Additionally, experimentally verified motifs enable the development of improved computer algorithms.

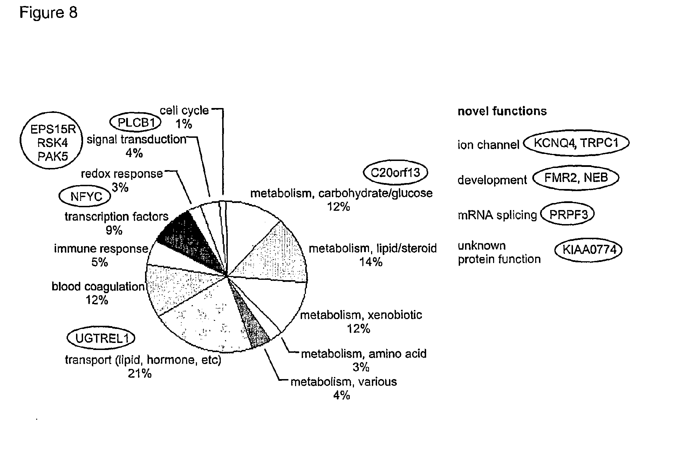

Next to ChIP-cloning of novel HNF4α gene targets identification may be achieved by studying gene expression after HNF4α overexpression. This approach yielded 62 novel gene candidates in a human hepatoma cell line (49) and 338 probe-sets (whereas approx. 50% of the probe-sets were annotated to specific genes) in a rat insulinoma cell line (70). In both studies HNF4α affects predominantly targets involved in metabolic processes, but targets involved in cell communication, cell cycle and development were also reported. However, no bioinformatic analyses or EMSA assays were applied to confirm findings. Moreover, the newly identified genes may not necessarily function as direct targets, because changes in gene expression might be due to indirect effects resulting from altered signal-transduction cascades.
Furthermore, HNF4α could influence gene expression by controlling HNF1α (44), PXR (pregnane X receptor) (34;44), PPARα (peroxisome proliferator-activated receptor α) (57), HNF6 (59) or other as yet unknown cooperating transcription factors. Therefore, regulated genes could, in part, be indirect targets, unless its binding was validated in *vivo.*
Finally, Odom et al (53) reported gene target identification for HNF1α, HNF4α, and HNF6 based on chromatin immunoprecipitation combined with DNA-DNA hybridization on a 13000 human promoter sequences containing microarray. In the case of HNF4α the number of contacted promoters was unexpected high, i.e. 1575 potential HNF4α target genes in hepatocytes were identified, corresponding to 12% of the genes represented on the array. Further, 42% of the genes occupied by RNA-polymerase II were also occupied by HNF4α. It was used the same antibody as reported by Odom et al (53). This antibody suffers from significant drawbacks in ChIP-experiments (see results) and yielded only low amounts of immunoprecipitated DNA. As reported above two consecutive rounds of immunoprecipitation in addition to n=3 independent experiments to confirm novel HNF4α candidate gene are performed. Additionally, it is considered a comparison of findings to the no antibody control as a must. Though it is undisputed that the ChIP-chip assay will be invaluable there is a definitive need for thorough evaluation of potential targets. Only 48 (= 3%) of 1575 putative HNF4α targets were verified in separate gene-specific ChIP experiments, though a 16% frequency of false positives was reported for the assay.
Additionally, HNF4α DNA-binding was not distinguished from protein-protein interactions, as in vitro binding was not analyzed. The potential targets remain speculative until they are carefully validated. When the data described by Naiki et al (49) were compared with data derived from the ChIP-chip assay only 17 genes were common. Likewise, when the novel HNF4α candidate genes from this report were compared with findings reported by Odom et al (53) only C20orf13 and UGTREL1 were in common, but none of the gene targets being identified were observed in the HNF4α overexpression studies reported by Naiki et al (49) and by Thomas et al (70).
In conclusion, this invention discloses the successful identification and validation of n = 13 HNF4α gene targets and suggest novel roles for this factor in cell cycle regulation, signal transduction, metabolism, nutrient transport, ion channel, development and mRNA splicing (see **Fig 8**). Therefore, HNF4α is versatile and functions beyond the control of metabolic processes. In the future, identification of genome wide targets regulated by a specific transcription factor may be feasible by coupling well-controlled ChIP-chip experiments with improved bioinformatic approaches, which allows for the development of composite modules that take combinatorial and synergistic action of several transcription factors into account.

### Methods

Experiments were performed using the following methods

### Caco-2 cell culture

Caco-2 cells were obtained from and cultivated as recommended by DSMZ. Essentially, cells were cultured in DMEM supplemented with 10% FCS and 200 µg/ml penicillin, 200 µg/ml streptomycin and 615 µg/ml L-glutamic acid. Cells were used between the 5. and the 20. passage and were checked for purity and morphological abnormalities by phase contrast microscopy. Caco-2 cells were seeded with a density of 4 x 10⁶ cells per 75 cm² flask and harvested after 11 days.

### Isolation of nuclear extracts

The use of animals was approved by the local government of Hannover with project license 02-548. Sprague Dawley rats (n=3) were treated with a single i.p. dose of 100 mg of Aroclor 1254 per kg bodyweight and killed 72 h later. Nuclear extracts from rat liver were prepared as described by Gorski et al (22), whereas nuclear extracts from Caco-2 cells were isolated by a modified method of Dignam et al (13). Eleven days after seeding cells were washed twice with ice-cold PBS, scraped into microcentrifuge tubes and centrifuged for 5 min at 2000 x g, 4°C. Cell pellets were resuspended in hypotonic buffer (10 mM Tris pH 7.4, 2 mM MgCl₂, 140 mM NaCl, 1 mM DTT, 4 mM Pefabloc, 1 % Aprotiniri, 40 mM β-glycerophosphate, 1 mM sodiumorthovanadate and 0.5% TX100) at 4°C for 10 min (300 µl for 1 x 10⁷ cells), transferred onto one volume of 50% sucrose in hypotonic buffer and centrifuged at 14000 x g and 4°C for 10 min. Nuclei were resuspended in Dignam C buffer (20 mM Hepes pH 7.9, 25% glycerol, 420 mM NaCl, 1.5 mM MlgCl₂, 0.2 mM EDTA, 1 mM DTT, 4 mM Pefabloc, 1% Aprotinin, 40 mM β-glycerophosphate, 1 mM sodiumorthovanadate, 30 µl for 1 x 10⁷ cells) and gently shaked at 4°C for 30 min. Nuclear debris was removed by centrifugation at 14000 x g at 4°C for 10 min. The extracts were aliquoted and stored at -70°C. Protein concentrations were determined according to the method of Smith et al (67).

### Western blot analysis

Nuclear extracts were separated on a 12% SDS-polyacrylamide gel and blotted onto a PVDF membrane in 25 mM Tris and 190 mM Glycin at 4°C for 3 h at 350 mA. The antibody directed against HNF4α was purchased from Santa Cruz Biotechnology (sc-8987x). The antigen-antibody complexes were visualized using the ECL detection system as recommended by the manufacturer NEN Life Science Products and chemiluminescence was recorded with Kodak IS 440 CF.

### Electrophoretic mobility shift assays

Nuclear extracts were used as described in the figure legends. Binding buffer consisted of 25 mM HEPES, pH 7.6, 5 mM MgCl₂, 34 mM KCI, 2 mM DTT, 2 mM Pefabloc, 2% Aprotinin, 40 ng of poly (dl-dC)/µl and 100 ng of bovine serum albumin/µl. Oligonucleotides and nuclear proteins were incubated for 20 minutes on ice. Free DNA and DNA-protein complexes were resolved on a 6% polyacrylamide gel. The oligonucleotides were purchased from MWG Biotech and used as ³²P-labeled probes, for sequence information see **Table 1**. Super shift experiments were done with antibodies (Santa Cruz Biotechnology) against HNF4α (sc-6556x), HNF1α (sc-6547x) or COUP-TF (sc-6578x).

### Crosslinking and Chromatin immunoprecipitation (ChIP)

All ChIP procedures were carried out as described by Weinmann et al (74) with some modifications. Caco-2 cells were treated with 1% formaldehyde at room temperature for 10 min under constant agitation. The reaction was stopped by the addition of glycine to obtain a final concentration of 125 mM. Cells were washed twice with ice-cold PBS, detached with trypsin and collected by centrifugation. Cells were resuspended in lysis buffer (5 mM PIPES, 85 mM KCI, 0.5% NP40 and 1x complete protease inhibitor, Roche) and incubated on ice for 20 min. The nuclei were collected by microcentrifugation and then resuspended in nuclei lysis buffer (1% SDS, 10 mM EDTA pH 8.0, 50 mM Tris-HCl pH 8.1 and 1x complete) and incubated on ice for 10 min. The samples were sonicated on ice until crosslinked chromatin was fragmented to approximately 0.2-1.6 kbp. Protein A-Sepharose CLB4 (Pharmacia) was blocked with 1 mg ml⁻¹ BSA and 1 mg ml⁻¹ herring sperm DNA (Promega) and washed extensively before use. Chromatin preparations were precleared by incubation with 'blocked' Protein A-Sepharose for 1 h at 4°C. The Protein A-Sepharose was removed by centrifugation and the precleared chromatin was diluted 1:3 with immunoprecipitation (IP) dilution buffer (0.01 % SDS, 1.2 mM EDTA pH 8.0, 1.1% Triton X100 and 1x complete). Precleared chromatin from 2.5x10⁷ cells was incubated with 1 µg goat polyclonal HNF4α antibody (sc-6556, Santa Cruz) or no antibody and rotated at 4°C overnight. Immunoprecipitates were recovered by incubation with a secondary antibody (rabbit anti-goat) and 'blocked' Protein A-Sepharose, washed twice with dialysis buffer (50 mM Tris-HCl pH 8.0, 2 mM EDTA, 0.2% sarkosyl) and four times with IP wash buffer (100 mM Tris-HCl pH 9.0, 500 mM LiCl, 1% NP40, 1% deoxycholic acid). Prior to the first wash, the supernatant from the reaction with no antibody was saved as total input chromatin and was processed with the eluted immunoprecipitates at the beginning of the cross-link reversal step. Elution was done with 30 µl elution buffer (1% SDS, 50 mM NaHCO₃) and samples were diluted 1:10 with IP dilution buffer. Two samples were pooled for a second immunoprecipitation step with the HNF4α antibody. After further recovering and washing steps, elution was done two times with 150 µl elution buffer each. Crosslinks were then reversed by the addition of NaCl to a final concentration of 300mM, and RNA was removed by the addition of 10 µg of RNase A per sample followed by incubation at 65°C for 4-5 h. The samples were then precipitated at -20°C overnight by addition of 2.5 volumes of ethanol and then pelleted by microcentrifugation. The samples were resuspended in 100 µl Tris-EDTA (pH 7.6), 25 µl 5x proteinase K buffer (1.25% SDS, 50 mM Tris-HCl pH 7.5, 25 mM EDTA pH 8.0) and 125 µg proteinase K (Roth) and incubated at 55°C for 2h. DNA purification was done by extraction with phenol-chloroform-isoamyl alcohol (25:24:1) and subsequent ethanol precipitation. The pellets were resuspended in 30 µl H₂O and analyzed by PCR. A mock probe, containing buffer without chromatin, was treated categorically throughout the whole immunoprecipitation procedure and throughout DNA isolation and purification to control for external DNA contamination.
PCR was done in a mixture containing 2 µl of purified DNA or 2 µl of a 1:200 dilution of the total input sample, 1 µM of each primer, 0.25 mM dNTP mixture, 0.625 U Thermostart-Taq (Abgene) and 1 x PCR-buffer (Abgene, with 1.5 mM MgCl₂) in a total volume of 20 µl. PCRs were carried out with a T3 Thermocycler (Biometra, Göttingen, Germany) with the following conditions: initial denaturation at 95°C for 15 min (Thermostart activation), denaturation at 94°C for 30 sec, annealing at different temperatures for 45 sec (**Table 2**), extension at 72°C for 45 sec, final extension at 74°C for 10 min, 45 cycles. A detailed account of PCR primers to analyze immunoprecipitated target genes is given in **Table 2**. The PCR-amplification products were run on a 2.0% agarose gel and analyzed by ethidium bromide staining.

### Cloning of immunoprecipitated DNA

The immunoprecipitated DNA was treated with T4 DNA polymerase (New England Biolabs) to create blunt ends, purified, and cloned into the zero-blunt vector (Invitrogen) using the zero-blunt PCR cloning kit (Invitrogen) according to the manufacturers recommendations. Colonies having inserts were identified by restriction enzyme digestion using enzymes in the polylinker.

### Sequence Analysis

Plasmid-DNA was purified with QIAquick PCR Purification Kit (Qiagen), subjected to cycle sequencing with vector-specific primers using BiaDyeTerminator v3.1 Kit and injected into ABI 3100 Genetic Analyzer (Applied Biosystems). Sequences were identified by database searches (GenBank version Build 35.1, maintained by NCBI) for human genomic matches. Detailed sequence information is given in Table 3.

### Bioinformatic searching for HNF4α binding-sites

The transcription start site (TSS, +1) of the NCBI mRNA reference sequence (RefSeq) was aligned using the UCSC Genome Browser (http://genome.ucsc.edu/) for promoter annotation of the respective clones. Cloned fragments and respective proximal promoters of gene targets (-1 to -3000 bp) were checked for putative HNF4α binding-sites with two different weight matrix-based tools, i.e. V$HNF4_01 with cut-off core similarity 0.75 and matrix similarity 0.78, Transfac matrix (Biobase, www.biobase.de) and V$HNF4_01 with cut-off core similarity 0.75 and matrix similarity 0.82 or V$HNF4_02 with cut-off core similarity 0.75 and matrix similarity 0.76, Genomatix matrix (Genomatix, www.genomatox.de).

### RT-PCR

Total RNA was isolated using the nucleospin RNA Isolation Kit (Macherey-Nagel) according to the manufacturers recommendations. 4 µg total RNA from each sample was used for reverse transcription (Omniscript Reverse Transcriptase, Qiagen). PCR was done in a mixture containing a cDNA equivalent to 25 ng of total RNA, 1 µM of each primer, 0.25 mM dNTP mixture, 0.625 U Thermostart-Taq (Abgene) and 1x PCR-buffer (Abgene, with 1.5 mM MgCl₂) in a total volume of 20 µl. PCR-reactions were carried out with a thermocycler (T3, Biometra) with the following conditions: initial denaturation at 95°C for 15 min (Thermostart activation), denaturation at 94°C for 30 sec, annealing at different temperatures for 45 sec (**Table 4**), extension at 72°C for 45 sec, final extension at 74°C for 10 min. Various cycle numbers were used to demonstrate linearity and amplification products were separated using a 1.5 % agarose gel and stained with ethidium bromide. A detailed oligonucleotide sequence information is given in **Table 4**.

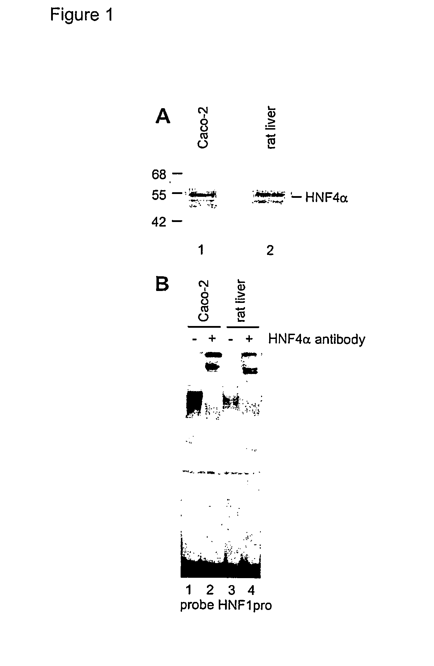

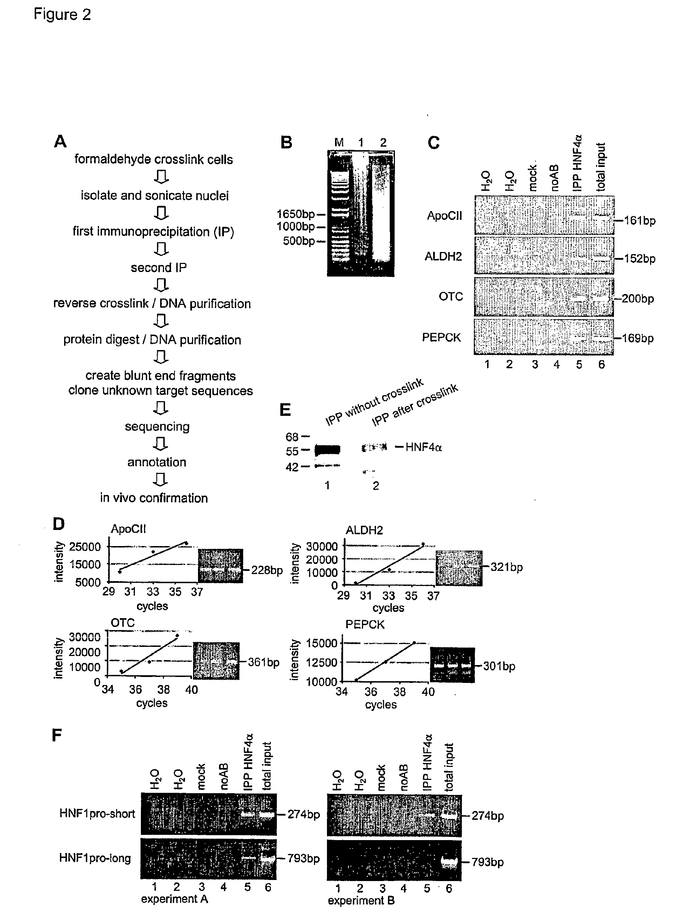

Based on formaldehyde crosslinking of nuclear proteins and cloning of immunoprecipitated DNA novel HNF4α gene targets were searched in the human intestinal cell line Caco-2, which differentiates spontaneously from cryptlike to villuslike enterocytes upon reaching confluence (29;55). At this stage, HNF4α protein expression is comparable to its expression in rat liver (Fig. 1A). Further, EMSA-studies provided additional evidence for abundant HNF4α DNA-binding to the A-site of the HNF1α-promoter (HNF1pro) (Fig. 1B), which is an established recognition site for HNF4α (63;66). The invention therefore discloses the ChIP-cloning (Fig. 2A) of novel HNF4α gene targets from total input DNA of highly differentiated Caco-2 cell cultures. After brief formaldehyde crosslinking nuclei were isolated and subsequently extracted as described in the Material and Methods section. Soluble chromatin was fragmented by mechanical shearing. The sonication of DNA was optimized to obtain DNA fragments between 200bp and 1600bp (Fig. 2B). The HNF4α immunoprecipitated DNA was screened for enrichment of promoter regions of well-known HNF4α target-genes and HNF4α recognition sites herein (63;66). PCR-assays of immunoprecipitated DNA enabled identification of apolipoprotein-CII (ApoCII), aldehyde-dehydrogenase-2 (ALDH2), ornithine-transcarbamylase (OTC) and phosphoenolpyruvate-carboxykinase (PEPCK), all of which are well known targets for HNF4α (Fig. 2C). To control for unspecific binding DNA was also prepared in the absence of the HNF4α antibody. As shown in Fig 2C, none of the requested and well-known HNF4α target sequences could be amplified. A "mock" sample containing buffer without chromatin was used throughout the immunoprecipitation procedure and throughout the DNA isolation to control for external DNA contamination derived from buffer and wash solutions. Finally, expression of the HNF4α targeted genes ApoCII, ALDH2, OTC and PEPCK was further confirmed by RT-PCR (Fig. 2D). The invention therefore provides strong evidence for immunoprecipitated DNA to be enriched for HNF4α binding sites and it demonstrates selectivity of the adopted procedure based on a well-controlled experimental approach. Noteworthy, two rounds were employed of consecutive chromatin immunoprecipitations to decrease the amount of nonspecific DNA. The price for high specificity was, however, low yield of DNA after the second immunoprecipitation step. Therefore, several immunoprecipitations were carried out in parallel and immunoprecipitated DNA was pooled following the DNA purification step (74;75). It is of considerable importance that single immunoprecipitations followed by PCR amplification of target sequences do not provide conclusive information for immunoprecipitates to contain target DNA. Indeed, signal enrichment of target sequences is insufficient when compared to the no antibody control. The procedure therefore requested two consecutive rounds of immunoprecipitation. Due to the second immunoprecipitation step, immunoprecipitated DNA yield was limited. Further, antibody recognition of the HNF4α epitope is partially hampered, when DNA is crosslinked (Fig. 1E). Presumably, masking of the epitope after formaldehyde crosslinking is a plausible reason for immunoprecipitated DNA not to contain the full complement of known HNF4α targets. The immunoprecipitated DNA was screened for established HNF4α targets by PCR and the assay was optimized to investigate the A-site within the HNF1α promoter. Different PCR strategies were used for smaller (274bp) and larger (793bp) amplification products. Strikingly, the long fragment (Fig. 1F) from HNF4α immunoprecipitated DNA could rarely be amplified, though immunoprecipitated DNA was positive for the short fragment. We assume DNA sonification to fragment DNA unfavorably and experimental conditions were standardized to obtain DNA fragments between 200bp and 1600bp and PCR assays were therefore optimized to allow for DNA template sizes between 150 and 300bp.

### ChIP cloning of novel HNF4α gene targets

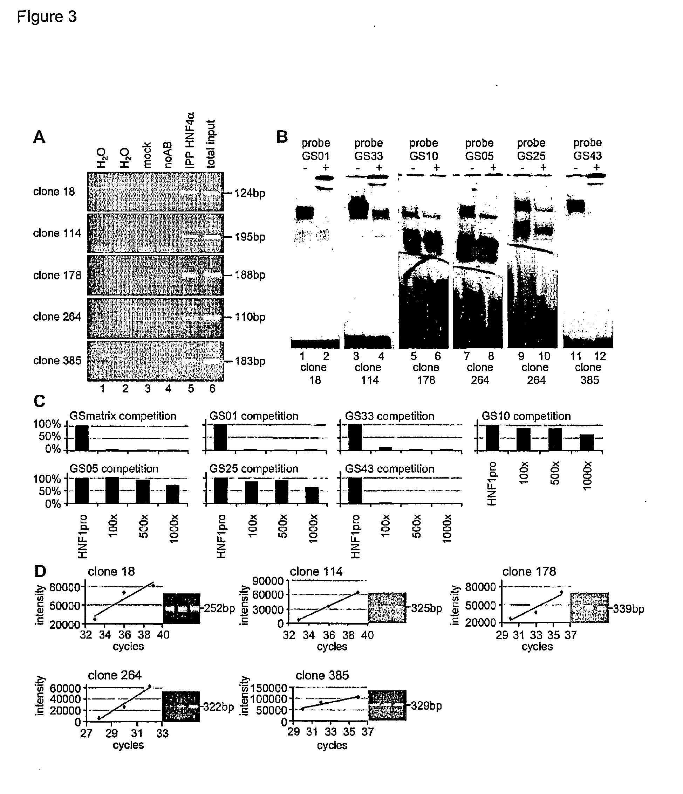

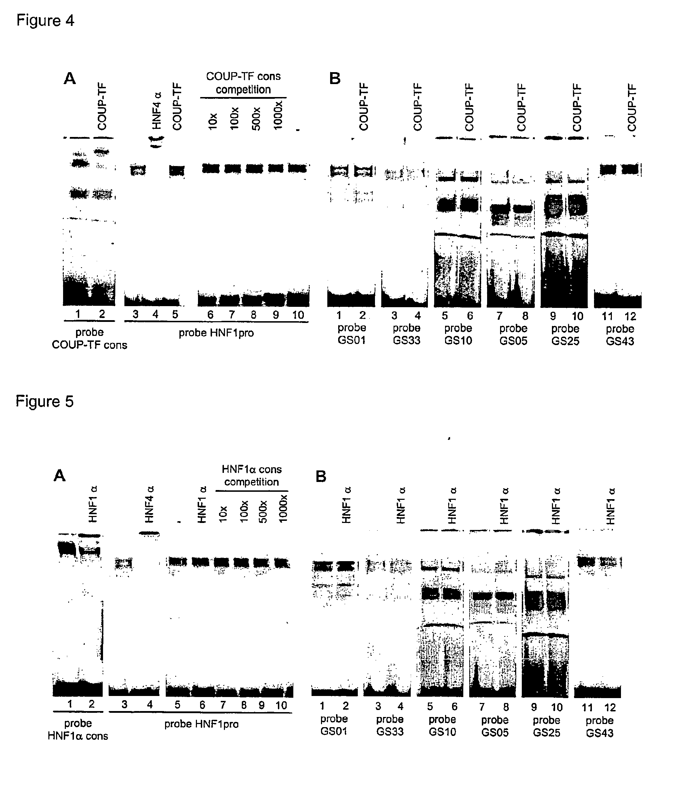

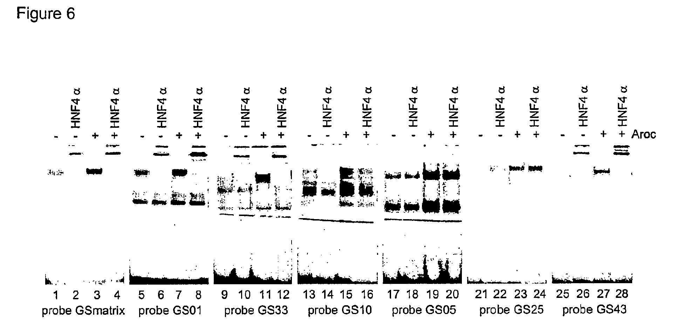

ChIP-assays of immunoprecipitated DNA yielded clones with inserts up to 1800bp. The inserts were sequenced by capillary electrophoresis and amplification with vector-specific primers and the genomic sequences were identified by database searches (GenBank, maintained by NCBI) for human genomic matches (Table 5). Approximately 50% of sequenced clones represented clearly annotated human sequences. Thereof, one half could be annotated within genes with established or predicted functions or were mapped to EST's, whereas the other half of clones could be traced back to known chromosomal localization but are of uncertain gene ID's. Nonetheless, these clones did harbor regulatory regions for HNF4α. In addition, some of the cloned fragments were within intronic regions and this agrees well with findings reported by others (23;46;68). Therefore proximal promoter sequences were analyzed. Cloned fragments as well as promoter sequences were interrogated for putative HNF4α binding-sites with two different bioinformatic matrixes. Accordingly, primer pairs were designed to confirm predicted sites experimentally and independent ChIP-experiments followed by PCR-analyses with clone-specific and/or promoter-specific primers enabled robust identification of novel HNF4α target genes.
The invention discloses the identification of 13 novel HNF4α gene targets in some detail. HNF4α in vivo binding was confirmed with clone 114 and clone 178 (Fig. 3A). Additionally, predicted binding-sites in the promoter of clone 18, clone 264 and clone 385 were bound specifically by HNF4α *in vivo* (Fig. 3A). The ability of HNF4α to bind to cognate recognition sites was studied by EMSA with ³²P-labeled probes specifically designed to encompass the predicted HNF4α-sites located in clone114 (GS33), clone178 (GS10), in the promoter of clone18 (GS01), clone264 (GS05, GS25) and clone385 (GS43) (Fig. 3B). Supershift experiments with a specific HNF4α antibody evidenced strong binding of HNF4α with the probes GS01, GS33 and GS43 but weaker binding with the probes GS05, GS10 and GS25. HNF4α displays different *in vitro* binding affinities for novel gene targets. To estimate binding affinity of HNF4α, competition experiments were carried out (Fig. 3C). The HNF1pro-site served as labeled probe to capture HNF4α nuclear protein and competition was first analyzed with a specifically designed probe based on a weighted bioinformatic matrix. This probe (GSmatrix) competed successfully for HNF4α binding (100x, reduction to 3,2%). Likewise, competitive EMSA with probes GS01, GS33 and GS43 provided evidence for strong binding with 100-fold excess of probes to result in 2,6%, 12,7% and 3% reduction in HNF4α binding, respectively, whereas competition with probe GS05, GS10 and GS25 was minimal, i.e. no change to approximately 85% at 100-fold excess. Competition experiments were complemented by supershift experiments, and in vivo and in vitro binding of HNF4α for clone114, clone178 and binding sites within the promoter of clone18, clone264 and clone385 were confirmed.
The invention further confirmed transcript expression of the new HNF4α gene targets (clone18, clone114, clone178, clone264 and clone385) in cultures of Caco-2 cells (Fig. 3D). This provided additional evidence for a role for HNF4α in the transcriptional regulation of these genes.
Further, the newly identified HNF4α binding sites were studied for interaction with COUP-TF or HNF1α. Initially it was assayed for COUP-TF (Fig. 4A, lane 1 and 2) and HNF1α (Fig. 5A, lane 1 and 2) and binding of these proteins was confirmed. Neither COUP-TF (Fig. 4A, lane 5) nor HNF1α (Fig. 5A, lane 4) contacted, however, the A-site of the HNF1α promoter (HNF1pro). Similar, COUP-TF (Fig. 4B) and HNF1α (Fig. 5B) did not bind to the newly identified HNF4α recognition sites.
As gene expression of a broad range of CYP isozymes is regulated by HNF4α (33) and treatment of hepatocytes with Aroclor 1254 led to simultaneous induction of HNF4α and several detoxifying enzymes (4), HNF4α-binding in liver nuclear extracts of control and Aroclor treated rats was investigated by EMSA. Binding of HNF4α to an optimized binding sites (GSmatrix) as well as to the newly identified binding sites (GS01, GS33, GS10, GS05 and GS43) was increased after Aroclor treatment (Fig. 6), thus providing additional evidence for the newly identified gene targets to be striktly regulated by HNF4α.
A summary of the cloned HNF4α targets is given in Table 6. Clone18 contained a ChIP-verified HNF4α binding site in the promoter region (around -2539) and was identified as C20orf13, a gene coding for a protein with predicted asparaginase activity. Clone114 was ChIP-verified from the first intron and identified as KIAA0774.
The function of the coded protein product is so far unknown. Clone178 was ChIP-verified from the first intron and identified as epidermal growth factor receptor substrate (EPS15R). Clone264 contained a ChIP-verified HNF4α binding site in the promoter region (around -928) and was identified as phospholipase C, beta 1 (PLCB1). Clone385 contained a ChIP-verified HNF4α binding site in the promoter region (around -3578) and was identified as UDP-galactose transporter related protein (UGTREL1). Additionally, two kinases, RSK4 (clone23) and PAK5 (clone113) were cloned and confirmed as novel HNF4α gene targets (data reported in detail elsewhere (51 )). Table 7 gives an account of HNF4α gene targets with confirmed in *vivo* binding (Fig. 7A) but lack HNF4α *in vitro* binding to the ChIP confirmed fragments as studied by EMSA (Fig. 7B). Further, transcript expression of these clones in Caco-2 cells was confirmed (Fig. 7C). Clone84 contained a ChIP-verified HNF4α binding site in the promoter region (around -575) and was identified as fragile X mental retardation protein 2 (FMR2). Clone177 contained a ChIP-verified HNF4α binding sites in the promoter region (around -355) and was identified as nebulin, i.e. a giant muscle protein. Clone261 was ChIP-verified from a region located around 7.5 kb upstream of potassium voltage-gated channel, KQT-like subfamily, member 4 (KCNQ4) and around 4.7 kb downstream of the C-subunit of nuclear factor Y (NFYC). Clone310 contained a ChIP-verified HNF4α binding site in the promoter region (around -5) and was identified as pre-mRNA processing factor 3 (PRPF3). Clone460 was ChIP-verified from the first intron and identified as transient receptor potential cation channel, subfamily C, member 1 (TRPC1). In addition, in vitro binding of HNF4α to further putative promoter binding sites was analyzed and HNF4α binding to FMR2, NFYC, KCNQ4 and PRPF3 was observed (Fig. 7D).

The discovery of the above described target genes of HNF4α and their function comprises an enormous potential for the treatment of metabolic diseases including diabetes and diabetic caused diseases and tumor growth. By cooperation with pharmaceutic companies this knowledge can be excellently used to develop new straightforward strategies and outstanding agents for the simple treatment of metabolic disorders. Such agents can be, for example, drugs which affect the genes or their gene products being dysregulated due to the disease.

Reference List
1. Antes, T. J., S. A. Goodart, W. Chen, and B. Levy-Wilson. 2001. Human apolipoprotein B gene intestinal control region. Biochemistry 40:6720-6730.
2. Barski, A. and B. Frenkel. 2004. ChIP Display: novel method for identification of genomic targets of transcription factors. Nucleic Acids Res 32:e104.
3. Bisaha, J. G., T. C. Simon, J. I. Gordon, and J. L. Breslow. 1995. Characterization of an Enhancer Element in the Human Apolipoprotein C-III Gene That Regulates Human Apolipoprotein A-I Gene Expression in the Intestinal Epithelium. J.Biol.Chem. 270:19979-19988.
4. Borlak, J. and T. Thum. 2001. Induction of nuclear transcription factors, cytochrome P450 monooxygenases, and glutathione S-transferase alpha gene expression in Aroclor 1254-treated rat hepatocyte cultures. Biochemical Pharmacology 61:145-153.
5. Caricasole, A., C. Sala, R. Roncarati, E. Formenti, and G. C. Terstappen. 2000. Cloning and characterization of the human phosphoinositide-specific phospholipase C-beta 1 (PLC beta 1). Biochim.Biophys.Acta 1517:63-72.
6. Chakarova, C. F., M. M. Hims, H. Bolz, L. Abu-Safieh, R. J. Patel, M. G. Papaioannou, C. F. Inglehearn, T. J. Keen, C. Willis, A. T. Moore, T. Rosenberg, A. R. Webster, A. C. Bird, A. Gal, D. Hunt, E. N. Vithana, and S. S. Bhattacharya. 2002. Mutations in HPRP3, a third member of pre-mRNA splicing factor genes, implicated in autosomal dominant retinitis pigmentosa. Hum Mol Genet 11:87-92.
7. Chen, W. S., K. Manova, D. C. Weinstein, S. A. Duncan, A. S. Plump, V. R. Prezioso, R. F. Bachvarova, and J. E. Darnell, Jr. 1994. Disruption of the HNF-4 gene, expressed in visceral endoderm, leads to cell death in embryonic ectoderm and impaired gastrulation of mouse embryos. Genes Dev 8:2466-2477.
8. Chiba, H., T. Gotoh, T. Kojima, S. Satohisa, K. Kikuchi, M. Osanai, and N. Sawada. 2003. Hepatocyte nuclear factor (HNF)-4alpha triggers formation of functional tight junctions and establishment of polarized epithelial morphology in F9 embryonal carcinoma cells. Exp Cell Res 286:288-297.
9. Chiba, H., T. Itoh, S. Satohisa, N. Sakai, H. Noguchi, M. Osanai, T. Kojima, and N. Sawada. 2005. Activation of p21 (CIP1/WAF1) gene expression and inhibition of cell proliferation by overexpression of hepatocyte nuclear factor-4alpha. Exp Cell Res 302:11-21.
10. Clapham, D. E. 2003. TRP channels as cellular sensors. Nature 426:517-524.
11. DeBelle, I., J. X. Wu, S. Sperandio, D. Mercola, and E. D. Adamson. 2003. In vivo cloning and characterization of a new growth suppressor protein TOE1 as a direct target gene of Egr1. J Biol Chem 278:14306-14312.
12. Deloukas, P., L. H. Matthews, J. Ashurst, J. Burton, J. G. Gilbert, M. Jones, G. Stavrides, J. P. Almeida, A. K. Babbage, C. L. Bagguley, J. Bailey, K. F. Barlow, K. N. Bates, L. M. Beard, D. M. Beare, O. P. Beasley, C. P. Bird, S. E. Blakey, A. M. Bridgeman, A. J. Brown, D. Buck, W. Burrill, A. P. Butler, C. Carder, N. P. Carter, J. C. Chapman, M. Clamp, G. Clark, L. N. Clark, S. Y. Clark, C. M. Clee, S. Clegg, V. E. Cobley, R. E. Collier, R. Connor, N. R. Corby, A. Coulson, G. J. Coville, R. Deadman, P. Dhami, M. Dunn, A. G. Ellington, J. A. Frankland, A. Fraser, L. French, P. Garner, D. V. Grafham, C. Griffiths, M. N. Griffiths, R. Gwilliam, R. E. Hall, S. Hammond, J. L. Harley, P. D. Heath, S. Ho, J. L. Holden, P. J. Howden, E. Huckle, A. R. Hunt, S. E. Hunt, K. Jekosch, C. M. Johnson, D. Johnson, M. P. Kay, A. M. Kimberley, A. King, A. Knights, G. K. Laird, S. Lawlor, M. H. Lehvaslaiho, M. Leversha, C. Lloyd, D. M. Lloyd, J. D. Lovell, V. L. Marsh, S. L. Martin, L. J. McConnachie, K. McLay, A. A. McMurray, S. Milne, D. Mistry, M. J. Moore, J. C. Mullikin, T. Nickerson, K. Oliver, A. Parker, R. Patel, T. A. Pearce, A.I. Peck, B. J. Phillimore, S. R. Prathalingam, R. W. Plumb, H. Ramsay, C. M. Rice, M. T. Ross, C. E. Scott, H. K. Sehra, R. Shownkeen, S. Sims, C. D. Skuce, M. L. Smith, C. Soderlund, C. A. Steward, J. E. Sulston, M. Swann, N. Sycamore, R. Taylor, L. Tee, D. W. Thomas, A. Thorpe, A. Tracey, A. C. Tromans, M. Vaudin, M. Wall, J. M. Wallis, S. L. Whitehead, P. Whittaker, D. L. Willey, L. Williams, S. A. Williams, L. Wilming, P. W. Wray, T. Hubbard, R. M. Durbin, D. R. Bentley, S. Beck, and J. Rogers. 2001. The DNA sequence and comparative analysis of human chromosome 20. Nature 414:865-871.
13. Dignam, J. D., R. M. Lebovitz, and R. G. Roeder. 1983. Accurate transcription initiation by RNA polymerase II in a soluble extract from isolated mammalian nuclei. Nucleic Acids Res 11:1475-1489.
14. Donner, K., M. Sandbacka, V. L. Lehtokari, C. Wallgren-Pettersson, and K. Pelin. 2004. Complete genomic structure of the human nebulin gene and identification of alternatively spliced transcripts. Eur.J Hum Genet 12:744-751.
15. Durmus, C., S. Yetiser, and O. Durmus. 2004. Auditory brainstem evoked responses in insulin-dependent (ID) and non-insulin-dependent (NID) diabetic subjects with normal hearing. Int J Audiol. 43:29-33.
16. Dusing, M. R., A. G. Brickner, S. Y. Lowe, M. B. Cohen, and D. A. Wiginton. 2000. A duodenum-specific enhancer regulates expression along three axes in the small intestine. Am J Physiol Gastrointest.Liver Physiol 279:G1080-G1093.
17. Ellrott, K., C. Yang, F. M. Sladek, and T. Jiang. 2002. Identifying transcription factor binding sites through Markov chain optimization. Bioinformatics. 18 Suppl 2 :S100-S109.
18. Faenza, I., A. Bavelloni, R. Fiume, G. Lattanzi, N. M. Maraldi, R. S. Gilmour, A. M. Martelli, P. G. Suh, A. M. Billi, and L. Cocco. 2003. Up-regulation of nuclear PLCbeta1 in myogenic differentiation. J Cell Physiol 195:446-452.
19. Fernandez, P. C., S. R. Frank, L. Wang, M. Schroeder, S. Liu, J. Greene, A. Cocito, and B. Amati. 2003. Genomic targets of the human c-Myc protein. Genes Dev 17:1115-1129.
20. Gailus-Durner, V., M. Scherf, and T. Werner. 2001. Experimental data of a single promoter can be used for in silico detection of genes with related regulation in the absence of sequence similarity. Mamm.Genome 12:67-72.
21. Galson, D. L., T. Tsuchiya, D. S. Tendler, L. E. Huang, Y. Ren, T. Ogura, and H. F. Bunn. 1995. The orphan receptor hepatic nuclear factor 4 functions as a transcriptional activator for tissue-specific and hypoxia-specific erythropoietin gene expression and is antagonized by EAR3/COUP-TF1. Mol.Cell.Biol. 15:2135-2144.
22. Gorski, K., M. Carneiro, and U. Schibler. 1986. Tissue-specific in vitro transcription from the mouse albumin promoter. Cell 47:767-776.
23. Greenbaum, S. and Y. Zhuang. 2002. Identification of E2A target genes in B lymphocyte development by using a gene tagging-based chromatin immunoprecipitation system. Proc Natl Acad Sci U.S.A 99:15030-15035.
24. Gregori, C., A. Porteu, C. Mitchell, A. Kahn, and A. L. Pichard. 2002. In vivo functional characterization of the aldolase B gene enhancer. J Biol Chem 277:28618-28623.
25. Gu, Y., K. L. McIlwain, E. J. Weeber, T. Yamagata, B. Xu, B. A. Antalffy, C. Reyes, L. Yuva-Paylor, D. Armstrong, H. Zoghbi, J. D. Sweatt, R. Paylor, and D. L. Nelson. 2002. Impaired conditioned fear and enhanced long-term potentiation in Fmr2 knock-out mice. J Neurosci. 22:2753-2763.
26. Guo, H., J. Wei, Y. Inoue, F. J. Gonzalez, and P. C. Kuo. 2003. Serine/threonine phosphorylation regulates HNF-4alpha-dependent redox-mediated iNOS expression in hepatocytes. Am J Physiol Cell Physiol 284:C1090-C1099.
27. Hatzis, P. and I. Talianidis. 2002. Dynamics of enhancer-promoter communication during differentiation-induced gene activation. Mol Cell 10:1467-1477.
28. Hayhurst, G. P., Y. H. Lee, G. Lambert, J. M. Ward, and F. J. Gonzalez. 2001. Hepatocyte nuclear factor 4alpha (nuclear receptor 2A1) is essential for maintenance of hepatic gene expression and lipid homeostasis. Mol Cell Biol 21:1393-1403.
29. Hu, C. and D. H. Perlmutter. 1999. Regulation of alpha1-antitrypsin gene expression in human intestinal epithelial cell line caco-2 by HNF-1alpha and HNF-4. Am J Physiol 276:G1181-G1194.
30. Hug, B. A., N. Ahmed, J. A. Robbins, and M. A. Lazar. 2004. A chromatin immunoprecipitation screen reveals protein kinase Cbeta as a direct RUNX1 target gene. J Biol Chem. 279:825-830.
31. Ishida, N., N. Miura, S. Yoshioka, and M. Kawakita. 1996. Molecular cloning and characterization of a novel isoform of the human UDP-galactose transporter, and of related complementary DNAs belonging to the nucleotide-sugar transporter gene family. J Biochem.(Tokyo) 120:1074-1078.
32. Jishage, M., T. Fujino, Y. Yamazaki, H. Kuroda, and T. Nakamura. 2003. Identification of target genes for EWS/ATF-1 chimeric transcription factor. Oncogene 22:41-49.
33. Jover, R., R. Bort, M. J. Gomez-Lechon, and J. V. Castell. 2001. Cytochrome P450 regulation by hepatocyte nuclear factor 4 in human hepatocytes: a study using adenovirus-mediated antisense targeting. Hepatology 33:668-675.
34. Kamiya, A., Y. Inoue, and F. J. Gonzalez. 2003. Role of the hepatocyte nuclear factor 4[alpha] in control of the pregnane X receptor during fetal liver development. Hepatology 37:1375-1384.
35. Kel, A., O. Kel-Margoulis, V. Babenko, and E. Wingender. 1999. Recognition of NFATp/AP-1 composite elements within genes induced upon the activation of immune cells. J Mol Biol 288:353-376.
36. Kel, A. E., O. V. Kel-Margoulis, P. J. Farnham, S. M. Bartley, E. Wingender, and M. Q. Zhang. 2001. Computer-assisted identification of cell cycle-related genes: new targets for E2F transcription factors. J Mol Biol 309:99-120.
37. Kim, J. H., P. Hui, D. Yue, J. Aycock, C. Leclerc, A. R. Bjoring, and A. S. Perkins. 1998. Identification of candidate target genes for EVI-1, a zinc finger oncoprotein, using a novel selection strategy. Oncogene 17:1527-1538.
38. Klapisz, E., I. Sorokina, S. Lemeer, M. Pijnenburg, A. J. Verkleij, and P. van Bergen en Henegouwen. 2002. A Ubiquitin-interacting Motif (UIM) Is Essential for Eps15 and Eps15R Ubiquitination. J.Biol.Chem. 277:30746-30753.
39. Klingenhoff, A., K. Frech, K. Quandt, and T. Werner. 1999. Functional promoter modules can be detected by formal models independent of overall nucleotide sequence similarity. Bioinformatics. 15:180-186.
40. Ktistaki, E. and I. Talianidis. 1997. Chicken ovalbumin upstream promoter transcription factors act as auxiliary cofactors for hepatocyte nuclear factor 4 and enhance hepatic gene expression. Mol Cell Biol 17:2790-2797.
41. Ktistaki, E. and I. Talianidis. 1997. Modulation of hepatic gene expression by hepatocyte nuclear factor 1. Science 277:109-112.
42. Le Hir, H., A. Nott, and M. J. Moore. 2003. How introns influence and enhance eukaryotic gene expression. Trends Biochem Sci 28:215-220.
43. Levine, M. and R. Tjian. 2003. Transcription regulation and animal diversity. Nature 424:147-151.
44. Li, J., G. Ning, and S. A. Duncan. 2000. Mammalian hepatocyte differentiation requires the transcription factor HNF-4alpha. Genes Dev 14:464-474**.**
45. Love-Gregory, L. D., J. Wasson, J. Ma, C. H. Jin, B. Glaser, B. K. Suarez, and M. A. Permutt. 2004. A Common Polymorphism in the Upstream Promoter Region of the Hepatocyte Nuclear Factor-4alpha Gene on Chromosome 20q Is Associated With Type 2 Diabetes and Appears to Contribute to the Evidence for Linkage in an Ashkenazi Jewish Population. Diabetes 53:1134-1140.
46. Martone, R., G. Euskirchen, P. Bertone, S. Hartman, T. E. Royce, N. M. Luscombe, J. L. Rinn, F. K. Nelson, P. Miller, M. Gerstein, S. Weissman, and M. Snyder. 2003. Distribution of NF-{kappa}B-binding sites across human chromosome 22. PNAS 100:12247-12252.
47. Matuoka, K. and C. K. Yu. 1999. Nuclear factor Y (NF-Y) and cellular senescence. Exp Cell Res 253:365-371.
48. Nagase, T., K. Ishikawa, M. Suyama, R. Kikuno, N. Miyajima, A. Tanaka, H. Kotani, N. Nomura, and O. Ohara. 1998. Prediction of the coding sequences of unidentified human genes. XI. The complete sequences of 100 new cDNA clones from brain which code for large proteins in vitro. DNA Res. 5:277-286.
49. Naiki, T., M. Nagaki, Y. Shidoji, H. Kojima, M. Imose, T. Kato, N. Ohishi, K. Yagi, and H. Moriwaki. 2002. Analysis of gene expression profile induced by hepatocyte nuclear factor 4alpha in hepatoma cells using an oligonucleotide microarray. J Biol Chem 277:14011-14019.
50. Nelson, E. A., S. R. Walker, J. V. Alvarez, and D. A. Frank. 2004. Isolation of unique STAT5 targets by chromatin immunoprecipitation-based gene identification. J Biol Chem.
51. Niehof, M. and J. Borlak. 2005. RSK4 and PAK5 are novel candidate genes in diabetic rat kidney and brain. Mol Pharmacol, Mol. Pharmacol. 67 604-611.
52. Nishiyori, A., H. Tashiro, A. Kimura, K. Akagi, K. Yamamura, M. Mori, and M. Takiguchi. 1994. Determination of tissue specificity of the enhancer by combinatorial operation of tissue-enriched transcription factors. Both HNF-4 and C/EBP beta are required for liver-specific activity of the ornithine transcarbamylase enhancer. J.Biol.Chem. 269:1323-1331.
53. Odom, D. T., N. Zizlsperger, D. B. Gordon, G. W. Bell, N. J. Rinaldi, H. L. Murray, T. L. Volkert, J. Schreiber, P. A. Rolfe, D. K. Gifford, E. Fraenkel, G. I. Bell, and R. A. Young. 2004. Control of Pancreas and Liver Gene Expression by HNF Transcription Factors. Science 303:1378-1381.
54. Parviz, F., C. Matullo, W. D. Garrison, L. Savatski, J. W. Adamson, G. Ning, K. H. Kaestner, J. M. Rossi, K. S. Zaret, and S. A. Duncan. 2003. Hepatocyte nuclear factor 4alpha controls the development of a hepatic epithelium and liver morphogenesis. Nat Genet 34:292-296.
55. Perlmutter, D. H., J. D. Daniels, H. S. Auerbach, K. Schryver-Kecskemeti, H. S. Winter, and D. H. Alpers. 1989. The alpha 1-antitrypsin gene is expressed in a human intestinal epithelial cell line. J.Biol.Chem. 264:9485-9490.
56. Phelps, D. E. and G. R. Dressier. 1996. Identification of novel Pax-2 binding sites by chromatin precipitation. J Biol Chem 271:7978-7985.
57. Pineda, T., I, Y. Jamshidi, D. M. Flavell, J. C. Fruchart, and B. Staels. 2002. Characterization of the human PPARalpha promoter: identification of a functional nuclear receptor response element. Mol Endocrinol. 16:1013-1028.
58. Qian, F., P. Huang, L. Ma, A. Kuznetsov, N. Tamarina, and L. H. Philipson. 2002. TRP genes: candidates for nonselective cation channels and store-operated channels in insulin-secreting cells. Diabetes 51 Suppl 1:S183-S189.
59. Rastegar, M., G. G. Rousseau, and F. P. Lemaigre. 2000. CCAAT/enhancer-binding protein-alpha is a component of the growth hormone-regulated network of liver transcription factors. Endocrinology 141:1686-1692.
60. Robinson, L., A. Panayiotakis, T. S. Papas, I. Kola, and A. Seth. 1997. ETS target genes: identification of egr1 as a target by RNA differential display and whole genome PCR techniques. Proc Natl Acad Sci U.S.A 94:7170-7175.
61. Romier, C., F. Cocchiarella, R. Mantovani, and D. Moras. 2003. The NF-YB/NF-YC structure gives insight into DNA binding and transcription regulation by CCAAT factor NF-Y. J Biol Chem. 278:1336-1345.
62. Santoro, R., S. Wolfl, and H. P. Saluz. 1999. UV-Laser induced protein/DNA crosslinking reveals sequence variations of DNA elements bound by c-Jun in vivo. Biochem Biophys Res Commun 256:68-74.
63. Schrem, H., J. Klempnauer, and J. Borlak. 2002. Liver-enriched transcription factors in liver function and development. Part I: the hepatocyte nuclear factor network and liver-specific gene expression. Pharmacol.Rev 54:129-158.
64. Seki, K. and A. Hata. 2004. Indian hedgehog gene is a target of the bone morphogenetic protein signaling pathway. J.Biol.Chem. 279:18544-18549.
65. Silander, K., K. L. Mohlke, L. J. Scott, E. C. Peck, P. Hollstein, A. D. Skol, A. U. Jackson, P. Deloukas, S. Hunt, G. Stavrides, P. S. Chines, M. R. Erdos, N. Narisu, K. N. Conneely, C. Li, T. E. Fingerlin, S. K. Dhanjal, T. T. Valle, R. N. Bergman, J. Tuomilehto, R. M. Watanabe, M. Boehnke, and F. S. Collins. 2004. Genetic Variation Near the Hepatocyte Nuclear Factor-4alpha Gene Predicts Susceptibility to Type 2 Diabetes. Diabetes 53:1141-1149.
66. Sladek, F. M. and S. D. Seidel . 2001. Hepatocyte nuclear factor 4alpha, p. 309-361. In T. Burris and E. R. B. McCabe (ed.), Nuclear Receptors and Disease. Academic Press, London.
67. Smith, P. K., R.I. Krohn, G. T. Hermanson, A. K. Mallia, F. H. Gartner, M. D. Provenzano, E. K. Fujimoto, N. M. Goeke, B. J. Olson, and D. C. Klenk. 1985. Measurement of protein using bicinchoninic acid. Anal.Biochem. 150:76-85.
68. Solano, P. J., B. Mugat, D. Martin, F. Girard, J. M. Huibant, C. Ferraz, B. Jacq, J. Demaille, and F. Maschat. 2003. Genome-wide identification of in vivo Drosophila Engrailed-binding DNA fragments and related target genes. Development 130:1243-1254.
69. Stevens, T. A., J. S. lacovoni, D. B. Edelman, and R. Meech. 2004. Identification of novel binding elements and gene targets for the homeodomain protein BARX2. J Biol Chem. 279:14520-14530.
70. Thomas, H., S. Senkel, S. Erdmann, T. Arndt, G. Turan, L. Klein-Hitpass, and G. U. Ryffel. 2004. Pattern of genes influenced by conditional expression of the transcription factors HNF6, HNF4alpha and HNF1beta in a pancreatic beta-cell line. Nucleic Acids Res 32:e150.
71. Tirona, R. G., W. Lee, B. F. Leake, L. B. Lan, C. B. Cline, V. Lamba, F. Parviz, S. A. Duncan, Y. Inoue, F. J. Gonzalez, E. G. Schuetz, and R. B. Kim. 2003. The orphan nuclear receptor HNF4alpha determines PXR- and CAR-mediated xenobiotic induction of CYP3A4. Nat Med 9:220-224.
72. Tsuchiya, T., Y. Kominato, and M. Ueda. 2002. Human hypoxic signal transduction through a signature motif in hepatocyte nuclear factor 4. J Biochem (Tokyo) 132:37-44.
73. Van Camp, G., P. J. Coucke, J. Akita, E. Fransen, S. Abe, E. M. De Leenheer, P. L. Huygen, C. W. Cremers, and S. Usami. 2002. A mutational hot spot in the KCNQ4 gene responsible for autosomal dominant hearing impairment. Hum Mutat 20:15-19.
74. Weinmann, A. S., S. M. Bartley, T. Zhang, M. Q. Zhang, and P. J. Farnham. 2001. Use of chromatin immunoprecipitation to clone novel E2F target promoters. Mol Cell Biol 21:6820-6832.
75. Weinmann, A. S. and P. J. Farnham. 2002. Identification of unknown target genes of human transcription factors using chromatin immunoprecipitation. Methods 26:37-47.

**Table 1. Shift-probes sequences**

| gene/clone | oligo-name | sequence |
|---|---|---|
| HNF1α | HNF1pro | AAGGCTGAAGTCCAAAGTTCAGTCCCTTC |
| - | GSmatrix | AGGGGGGGTCAAAGGTCACGGTC |
| - | HNF1cons | CCAGTTAATGATTAACCACTGGC |
| - | COUP-TF cons | TGAGCCCTTGACCCCT |
| clone 18, pro-site | GS01 | TTAGAGTACAAAGATCAAGATGC |
| clone 23, pro-site a | GS26 | AATGGAGGGCATAGGTCAACAGC |
| clone 23, pro-site b | GS27 | CCAGCGCTCAAAAGGTTGGCAGT |
| clone 84, pro-site a | GS28 | TCCAGGGCCTATAGCTCGCTGAC |
| clone 84, pro-site b | GS65 | CTGAAGGCATAAAGGTCGGGGGC |
| clone 113, site a | GS09 | TGTTGGGTACAATGTTCAATATT |
| clone 113, site b | GS16 | AAAGCTGACTAAGGTACATGTGC |
| clone 113, pro-site b | GS46 | TTAAGTGATTAAAGTTCAATATT |
| clone 114 | GS33 | AAATAGATACAAAGTCCTCCTTC |
| clone 177, pro-site a | GS24 | CGGAAGGGTTAAAGCCTCTCAAA |
| clone 177, pro-site b | GS03 | TGACTGGACAAATGACATGAAGA |
| clone 178 | GS10 | AACTGTGGTCCAAGCACACCATG |
| clone 261 | GS36 | CCTAGCTTTTGTCCCACAGCTCC |
| clone 261.1, pro-site | GS76 | GGGCGGGGTCAAAACTCAGATCT |
| clone 261.2, pro-site | GS82 | TGTGGTGGGGAAAGTCCGTCCTC |
| clone 264, pro-site a | GS05 | AAACCCGGGCAAACGGTTACGTT |
| clone 264, pro-site b | GS25 | CCCGGGTTTCAAGATCAGCGCGC |
| clone 310, pro-site a | GS23 | TAGGGCGGTCAGAAGGTTTCCGG |
| clone 310, pro-site b | GS70 | CCTGAGGTCCAGAGTTCGAGACG |
| clone 385, pro-site | GS43 | TGACAAGAACAAAGTCCATTTCA |
| clone 460, site a | GS34 | CTCACTGTGCAATGGTTATTTTA |
| clone 460, site b | GS35 | TCTTCCCTCAAAAGTTCTGAGAG |

**Table 2. ChIP-PCR primer sequences**

| gene / clone | primer name | primer sequence | fragment length | annealing |
|---|---|---|---|---|
| HNF1α | Ni16 | fwd: CATGATGCCCCTACAAGGTT rev:ATTGGAGCTGGGGAAATTCT | 274bp | 60°C |
| HNF1α | Ni18 | fwd:CAGCACTGTTCTTGGCACAT rev: CAGCACTGTTCTTGGCACAT | 793bp | 60°C |
| ApoCII | Ni57 | fwd:GTTCCCTGTGACGTGACCTT rev: ACGGGCACAGAGAGGATTTA | 161bp | 60°C |
| ALDH2 | Ni58 | fwd:CATCTCCTTCACCTCCGAAA rev: CAGCTCGCCTTGGTTGAG | 152bp | 60°C |
| OTC | Ni118 | fwd:AGGAGGCCAGGCAATAAAAG rev: GGGGGCCACCTAAAAACTAA | 200bp | 60°C |
| PEPCK | Ni119 | fwd:GGCACAGAGCAGACAATCAA rev: TTGGCAAAACACCACAGCTA | 169bp | 60°C |
| clone 18, pro-site | Ni3 | fwd:TCTTCCTGTTCCCACCTCTC rev: AGGACAGAGGGGGCTTACTT | 124bp | 60°C |
| clone 23, pro-site a | Ni8 | fwd:CCAACTCAGGACCTTGGAGA rev: GGCCAGCTTTGCTTCATTAG | 141bp | 60°C |
| clone 23, pro-site b | Ni13 | fwd: GAGCTGCTGTGCCTGGTACT rev: TTTTTGCTGACGGGAGAGAT | 148bp | 60°C |
| clone 84, pro-site a | Ni2 | fwd: CAAGGGCAGTCATTTGTTCC rev: GAAGGCGGTCACCTTCAC | 148bp | 60°C |
| clone 113, site a | Ni61 | fwd:TCATCACGGACATAAAGATGGA rev: GCATAGTGGTGGGGGTTCT | 160bp | 60°C |
| clone 113, site b | Ni73 | fwd:AGCAGAACCCCCACCACTAT rev: TCACCCAGAAAGTTCCCTTG | 187bp | 60°C |
| clone 113, pro-site a | Ni5 | fwd:CCGGTCAAGTCTGAACCAGT rev: TTAACTAGGCAAGCCCAAGC | 126bp | 60°C |
| clone 113, pro-site c | Ni116 | fwd:TAGTCCCTGTGGCTGCAGTA rev:TCTCAATGGCTGATTACAGGTT | 232bp | 60°C |
| clone 114 | Ni62 | fwd:AAAGGCCTAATCTTTTGTTTCTACA rev:TCACAGCACATTTTATGTGTCAA | 195bp | 60°C |
| clone 177, pro-site a and b | Ni115 | fwd:ACTTCCAGCCTGTGCAGTTC rev:CTGGTGAACCTTACCAGAGTGA | 196bp | 60°C |
| clone 178 | Ni64 | fwd:TGTCACTGCTCCAAACTGGT rev: ACCTTTGAGGTTTGGCCTTT | 188bp | 60°C |
| clone 261 | Ni88 | fwd:CCCTTCCCACCAACTCTTG rev: GAAGACACCAGCAGCCTAGC | 190bp | 60°C |
| clone 264, pro-site a and b | Ni7 | fwd:TCTGTCTCGAAAGCACAACG rev:AGAAGAGCGCAGTTGAGAGG | 110bp | 60°C |
| clone 310, pro-site | Ni9 | fwd:CTCCTTGGTCACGTGTTGG rev: CAAACTTCAGCCCCTGAGAC | 131bp | 60°C |
| clone 385, pro-site | Ni112 | fwd:AAGAGGGGCTTCATCAGGTT rev: CTCACCCTCTCTCGCTGTCT | 183bp | 60°C |
| clone 460, site a and b | Ni82 | fwd:TGGGTTGCCTGAGTTCTCTT rev:TCTGTCCTCAGTTTGACAGGAA | 182bp | 60°C |

**Table 3. Sequence information of clones**

| clone | gene name | Acc Number | chromosome | bp relative to transcription start site NCBl GenBank Version Build 35.1 |
|---|---|---|---|---|
| clone 18 | C20orf13 | NM_017714 | 20 | +198113 bis +198612 (500 bp) |
| clone 23 | RSK4 / RPS6KA6 | NM_014496 | X | +13766 to +14366 (601 bp) |
| clone 84 | FMR2 | NM_002025 | X | +8271 bis +8440 (170 bp) |
| clone 113 | PAK5/PAK7 | NM_045653 | 20 | +246492 to +246837 (346 bp) |
| clone114 | KIAA0774 | X_166270 | 13 | +7954 bis +8111 (158 bp) |
| clone177 | NEB | NM_004543 | 2 | +3957 bis +4396 (440 bp) |
| clone178 | EPS15R (EPS15L1) | NM_021235 | 19 | -7250 bis -7612 (363 bp) UCSC_hg17* +22121 bis +22483 (363 bp) |
| clone 261 | NFYC / KCNQ4 | NM_0142237 / NM_004700 | 1 | NFYC: +84624 bis +84750 (127bp) KCNQ4: -7694 bis -7568 (127bp) |
| clone 264 | PLCB1 | NM_015192 | 20 | +106006 bis +106181 (176 bp) |
| clone 310 | PRPF3 (HPRP3, PRP3) | NM_004698 | 1 | +16434 bis +16480 (47 bp) |
| clone 385 | UGTREL1 (SLC35B1) | NM_005827 | 17 | +6820 bis +6924 (105 bp) |
| clone 460 | TRPC1 | NM_003304 | 3 | +1632bis +1965(334bp) |

| | | | | |
|---|---|---|---|---|
| *UCSC Genome Browser, Version hg17 (http://genome.ucsc.edu/) | | | | |

**Table 4. RT-PCR primer sequences**

| gene / clone | primer name | accession number | primer | fragment length | annealing |
|---|---|---|---|---|---|
| ApoCll | Ni95 | NM_000483 | fwd:CCTCCCAGCTCTGTTTCTTG | 228bp | 60°C |
| | | | rev: GCTGCTGTGCTTTTGCTGTA | | |
| ALDH2 | Ni25 | NM_000690 | fwd:TGAAGGGGACAAGGAAGATG | 321bp | 58°C |
| | | | rev: ACAGGTTCATGGCGTGTGTA | | |
| OTC | Ni120 | NM_000531 | fwd:CATGGCAGATGCAGTATTGG | 261bp | 60°C |
| | | | rev: GGAGTAGCTGCCTGAAGGTG | | |
| PEPCK | Ni121 | NM_002591 | fwd:TCAGGCGGCTGAAGAAGTAT | 301bp | 60°C |
| | | | rev:ACGTAGGGTGAATCCGTCAG | | |
| clone18 | Ni29 | NM_017714 | fwd:CAAATGCAGGAATGGGATCT | 252bp | 58°C |
| | | | rev: GGCAAGAGGGTATTCCATGA | | |
| clone 23 | Ni69 | NM_014496 | fwd: GGATTTTCTCAGGGGAGGAG | 311bp | 60°C |
| | | | rev: AATCAGCACTCTGGGAATGG | | |
| clone 84 | Ni96 | NM_002025 | fwd:CACAAGGAGACTGCCACAAA | 328 bp | 50°C |
| | | | rev:AGGGACCATTATTGCCACTG | | |
| clone 113 | Ni72 | NM_020341 | fwd:GAATCAGACAAGCCCTCAGC | 309bp | 55°C |
| | | | rev: CCAGACGGGTACTGGTGACT | | |
| clone114 | Ni122 | X_166270 | fwd:CTGACCTTCCAGAGCCAGTC | 325bp | 50°C |
| | | | rev:TAGCATTTTCCTCCGACAGC | | |
| clone 177 | Ni51 | NM_004543 | fwd:CCCAGAGGCTACACCACAAT | 332 bp | 55°C |
| | | | rev: GAAAGCTTGCAACCCTTGAG | | |
| clone178 | Ni71 | NM_021235 | fwd: GCAGACAAGATGCGATTTGA | 339bp | 60°C |
| | | | rev:AAGCTCCTTCACGCCAGTAA | | |
| clone 261.1 | Ni102 | NFYC NM_0142237 | fwd:AAAGACTTCCGAGTGCAGGA | 316bp | 60°C |
| | | | rev: GCTCGGCAGGAGTTACAGAC | | |
| clone 261.2 | Ni103 | KCNQ4 NM_004700 | fwd:AGGAACTTGCCAACGAGTGT | 331bp | 60°C |
| | | | rev: CTATGCGCGTAGACCACTGA | | |
| clone264 | Ni52 | NM_015192 | fwd:GTTTTCAGCAGATCGGAAGC | 322bp | 55°C |
| | | | rev: GAGGCTGTTGTTGGGTTCAT | | |
| clone 310 | Ni53 | NM_004698 | fwd: CCCCAATGGCTTTGATCTTA | 321bp | 55°C |
| | | | rev: GCTCTGACGTGGGCTTCTAC | | |
| clone385 | Ni105 | NM_005827 | fwd:CTATCTGGGTGCCATGGTCT | 329bp | 60°C |
| | | | rev: GGTTGGAGCCTGTTTGGTAA | | |
| clone 460 | Ni106 | NM_003304 | fwd:TGGATGTTGCACCTGTCATT | 325bp | 54°C |
| | | | rev: TTACATTGCCGGGCTAGTTC | | |

**Table 5. Summary of ChIP clone annotations after sequencing.**

| | % of clones |
|---|---|
| total | 100 |
| cloning artefacts | 22.7 |
| total human sequences | 77.3 |
| total human sequences without DNA homology | 68.0 |
| not clearly identified | 14.7 |
| gene products with established or predicted function | 17.3 |
| ESTs, computer prediction, etc | 12.0 |
| DNA of known chromosomal location but uncertain gene ID and/or | 24.0 |
| function, in part putative regulatory regions | |

**Table 6. Summary of clone information with HNF4a in vitro binding to ChIP confirmed fragments.**

| clone | gene name | localization | | Swiss-Prot/ mRNA NCBI | mRNA expression in Caco-2 cells | HNF4α | promoter binding sites | molecular function | biological process |
|---|---|---|---|---|---|---|---|---|---|
| 18 | C20orf13 | chromosome | 20, 11. intron | Q9H6P5/ NM_017714 | yes | -2520 | CHIP confirmed EMSA binding | asparaginase activity (prediction) | metabolism / glycoprotein catabolism (prediction) |
| 114 | KLAA0774 | chromosome | 13, 1. intron CHIP confirmed EMSA binding | 094872/ XM_166270 | yes | | | hypothetical protein | unknown |
| 178 | EPS15R (EPS15L1) | chromosome | 19, 1. intron* CHIP confirmed EMSA binding | Q9UBC2/ NM_021235 | yes | | | receptor activity | endocytosis signal transduction |
| 264 | PLCB1 | chromosome | 20, 2. intron | Q9NQ66 / NM_015192 | yes | -919/-906 -919 -906 | CHIP confirmed EMSA binding site a EMSA binding site b | phospholipase activity | cell communication / signal transduction regulation of cell cycle |
| 385 | UGTREl1 (SLC35B1) | chromosome | 17, 3'UTR | P78383/ NM_005827 | yes | -3579 | CHIP confirmed EMSA binding | UDP-galactose transporter activity | transport |
| reported in detail in (51) | | | | | | | | | |
| 23 | RSK4 (RPS6KA6) | chromosome | X, 1.intron | Q9UK32/ NM_0144496 | yes | -1430 -2053 | CHIP confirmed EMSA binding ChIP confirmed EMSA binding | protein kinase activity | cell communication / signal transduction |
| 113 | PAK5 (PAK7) | chromosome | 20, 3. intron CHIP confirmed EMSA binding | Q9P2861 / NM_045653 | below the limit of detection | -951 -1766 -2181 | CHIP confirmed EMSA binding CHIP confirmed | protein kinase activity | cell communication / signal transduction |

| | | | | | | | | | |
|---|---|---|---|---|---|---|---|---|---|
| *UCSB Genome Browser, Version hgI7 (http://genome.ucsc.edu/).UCSC_hg17 = 1. intron /NCBI Version Build 35.1, NCBI b35.1=-7.5kb upstream TSS (see Supplement Table 3) | | | | | | | | | |

**Table 7. Summary of clone information without HNF4α in vitro binding to ChIP confirmed fragments.**

| clone | gene name T | | Swiss-Prot / mRNA NCBI | mRNA expression in Caco-2 cells | HNF4α | promoter binding sites | molecular function | biological process |
|---|---|---|---|---|---|---|---|---|
| 84 | FMR2 | chromosome X, 1. intron | P51816/ NM_002025 | yes | -576 -1893 | CHIP confirmed EMSA binding | transcription regulator activity (prediction) | development |
| 177 | NEB | chromosome 2, 4. intron | P20929/ NM_004543 | yes | -374/-341 | CHIP confirmed | structural constituent of muscle | development |
| 261 | | chromosome 1 ChIP confirmed | | | | | | |
| 261.1 | NFYC | 4.7 kb downstream | Q13952/ NM_014223 | yes | -163 | EMSA binding | transcription regulator activity | transcription / redoxresponse |
| 261.2 | KCNQ4 | 7,5 kb upstream | P56696/ NM_004700 | yes | -2518 | EMSA binding | ion channel activity | ion transport / ion channel |
| 310 | PRPF3 (HPRP3, PRP3) | chromosome 1, 9. intron | O43395/ NM_004698 | yes | -28 -3119 | CHIP confirmed EMSA binding | mRNA splicing factor activity | nuclear mRNA splicing |
| 460 | TRPC1 | chromosome 3, 1. intron CHIP confirmed | P48995 / NM_003304 | yes | | | ion channel activity | ion transport / ion channel |

### Figure Legends

### Figure 1

### HNF4α protein expression in differentiated Caco-2 cells.

(**A**) HNF4α western blotting analysis of 30 µg Caco-2 cell nuclear extract (lane 1) or 30 µg rat liver nuclear extract (lane 2). (**B**) Electrophoretic mobility shift experiment with 2,5 µg Caco-2 cell nuclear extract (lane 1 and 2) or 2,5 µg rat liver nuclear extract (lane 3 and 4) and an oligonucleotide corresponding to the A-site of the HNF1α promoter (HNF1pro) as ³²P labeled probe. For supershift analysis an antibody directed against HNF4α was added (lane 2, lane 4).

### Figure 2

### HNF4α chromatin immunoprecipitation assay with HNF4α target genes.

(**A**) Flow chart of HNF4α chromatin immunoprecipitation assay (ChIP-assay). (**B**) Fragmentation of total input DNA prior to (lane 1) and after sonification (lane 2) under standard conditions. (**C**) CHIP experiments were performed with cultures of Caco-2 cells and an antibody against HNF4α (IPP HNF4α, lane 5) or no antibody (noAB, lane 4). Following DNA purification samples were subjected to PCR with primers designed to amplify promoters of different HNF4α positive targets. The primers annealed proximal to the HNF4α binding-sites of the apolipoprotein CII (ApoCII) promoter, the aldehyde dehydrogenase 2 (ALDH2) promoter, the ornithine transcarbamylase (OTC) promoter, and the phosphoenolpyruvate carboxykinase (PEPCK) promoter, all of which are well-known HNF4α targets. A mock probe (mock, lane 3) and an aliquot of the total input sample (total input, lane 6) were also examined by PCR. Routinely, two reactions containing H₂O instead of template were included in each PCR as negative control (lane 1 and 2). (**D**) Gene expression of ApoCII, ALDH2, OTC and PEPCK in cultures of Caco-2 cells was analyzed by RT-PCR. A linear range of amplification cycles was shown. (**E**) HNF4α immunoprecipitation with and without crosslink. HNF4α western blot analysis of HNF4α immunoprecipitated complexes of Caco-2 cells without crosslink (lane 1) or after crosslink (lane 2) with 1% formaldehyde. Prior to SDS-PAGE, crosslink samples were heated to 95 °C in conventional SDS-polyacrylamide gel electrophoresis gel-loading buffer in the presence of 0.5 M 2-mercaptoethanol for 1 h to reverse protein-protein crosslinks. (**F**) Comparison of short- and long-fragment ChIP-PCR. CHIP experiments were performed with cultures of Caco-2 cells and an antibody against HNF4α (IPP HNF4α, lane 5) or no antibody (noAB, lane 4). Following DNA purification samples were subjected to PCR with primers designed to amplify short (274bp) or long fragments (793bp) containing the A-site within the HNF1α promoter as HNF4α positive target. A mock probe (mock, lane 3) and an aliquot of the total input sample (total input, lane 6) were also examined by PCR. Routinely two reactions containing H₂O instead of template were included in each PCR as negative control (lane 1 and 2). Two experiments were shown exemplarily.

### Figure 3

### Confirmation of CHIP clones by examination of HNF4α binding in vivo und in vitro, corresponding to Table 6.

(**A**) Independent CHIP experiments were performed with cultures of Caco-2 cells and an antibody against HNF4α (IPP HNF4α, lane 5) or no antibody (noAB, lane 4). Following DNA purification samples were subjected to PCR with primers designed for putative HNF4α binding-sites of clones and their promoters (clone18, clone114, clone178, clone264, clone385). A mock probe (lane 3) and an aliquot of the total input sample (lane 6) were also examined by PCR. Routinely two reactions containing H₂O instead of template were included in each PCR as negative control (lane 1 and 2). (**B**) Electrophoretic mobility shift assays with 2,5 µg Caco-2 cell nuclear extract and oligonucleotides (GS01, lane 1 and 2; GS33, lane 3 and 4; GS10, lane 5 and 6; GS05, lane 7 and 8; GS25, lane 9 and 10; GS43, lane 11 and 12) corresponding to putative HNF4α binding-sites within the identified clones and promoters as ³²P labeled probe. In supershift assays an antibody directed against HNF4α (+) was added (lane 2, 4, 6, 8, 10, 12). (**C**) Competition experiments. Electrophoretic mobility shift experiments were carried out with 2,5 µg Caco-2 cell nuclear extracts and an oligonucleotide corresponding to the A-site of the HNF1α promoter as ³²P labeled probe. An optimized HNF4α binding-site (GSmatrix) and the putative HNF4α binding-sites (GS01, GS33, GS10, GS05, GS25, GS43) were added as 100 fold, 500 fold and 1000 fold molar excess. Dried gels were analysed with a Molecular Imager (BioRad) using the Quantity One software (BioRad). HNF4α binding to the A-site of the HNF1α promoter as ³²P labeled probe was set to 100% and competition was quantified for each oligonucleotide. (**D**) Gene expression of clone 18, clone 114, clone 178, clone264 and clone385 in cultures of Caco-2 cells was analyzed by RT-PCR. A linear range of amplification cycles was shown.

### Figure 4

### Analysis of COUP-TF interference in HNF4a DNA binding.

(A) Electrophoretic mobility shift assays with 10 µg Caco-2 cell nuclear extract and an oligonucleotide corresponding to a consensus COUP-TF binding-site (COUP-TF cons, lane 1 and 2) or 2,5 µg Caco-2 cell nuclear extract and an oligonucleotide corresponding to the A-site of the HNF1α promoter (HNF1pro, lane 3 to 10) as ³²P labeled probe. For supershift analysis antibodies directed against COUP-TF (lane 2 and 5) and against HNF4α (lane 4) were added. (**B**) Electrophoretic mobility shift assays with 10 pg Caco-2 cell nuclear extract and oligonucleotides corresponding to the putative HNF4α binding-sites (GS01, lane 1 and 2; GS33, lane 3 and 4; GS10, lane 5 and 6; GS05, lane 7 and 8; GS25, lane 9 and 10; GS43, lane 11 and 12) as ³²P labeled probe. For supershift analysis an antibody directed against COUP-TF (lane 2, 4, 6, 8, 10, 12) was added.

### Figure 5

### Analysis of HNF1α interference in HNF4α DNA binding.

(A) Electrophoretic mobility shift assays with 10 µg Caco-2 cell nuclear extract and an oligonucleotide corresponding to a consensus HNF1α binding-site (HNF1α cons, lane 1 and 2) or 2,5 µg Caco-2 cell nuclear extract and an oligonucleotide corresponding to the A-site of the HNF1α promoter (HNF1pro, lane 3 to 10) as ³²P labeled probe. For supershift analysis antibodies directed against HNF1α (lane 2 and 6) and against HNF4α (lane 4) were added. (**B**) Electrophoretic mobility shift assays with 10 µg Caco-2 cell nuclear extract and oligonucleotides corresponding to the putative HNF4α binding-sites (GS01, lane 1 and 2; GS33, lane 3 and 4; GS10, lane 5 and 6; GS05, lane 7 and 8; GS25, lane 9 and 10; GS43, lane 11 and 12) as ³²P labeled probe. For supershift analysis an antibody directed against HNF1α (lane 2, 4, 6, 8, 10, 12) was added.

### Figure 6

### Aroclor treatment induces HNF4α in rat liver: in vitro binding to new targets

Electrophoretic mobility shift assay with 2,5 µg rat liver nuclear extract of control (lane 1, 2, 5, 6, 9, 10, 13, 14, 17, 18, 21, 22, 25, 26) or Aroclor treated animals (lane 3, 4, 7, 8, 11, 12, 15, 16, 19, 20, 23, 24, 27, 28) and oligonucleotides corresponding to putative HNF4α binding-sites (GSmatrix, lane 1-4; GS01, lane 5-8; GS33, lane 9-12; GS10, lane 13-16; GS05, lane 17-20; GS25, lane 21-24; GS43, lane 25-28) as ³²P labeled probe. For supershift analysis an antibody directed against HNF4α was added (lane 2, 4, 6, 8, 10, 12, 14, 16, 18, 20, 22, 24, 26, 28).

### Figure 7

### Confirmation of CHIP clones by examination of HNF4α binding in vivo und in vitro, corresponding to Table 7.

(**A**) CHIP experiments were performed with cultures of Caco-2 cells and an antibody against HNF4α (IPP HNF4α, lane 5) or no antibody (noAB, lane 4). Following DNA purification samples were subjected to PCR with primers designed for putative HNF4α binding-sites of clones and their promoters (clone84, clone177, clone261, clone310, clone460). A mock probe (lane 3) and an aliquot of the total input sample (lane 6) were also examined by PCR. Routinely two reactions containing H₂O instead of template were included in each PCR as negative control (lane 1 and 2). (**B**) Analysis of HNF4α *in vitro* binding to *in vivo* confirmed fragments. Electrophoretic mobility shift assays with 2,5 µg Caco-2 cell nuclear extract and oligonucleotides (GS28, lane 1 and 2; GS03, lane 3 and 4; GS24, lane 5 and 6; GS36, lane 7 and 8; GS23, lane 9 and 10; GS34, lane 11 and 12; GS35, lane 13 and 14) corresponding to putative HNF4α binding-sites as ³²P labeled probe. In supershift assays an antibody directed against HNF4α (+) was added (lane 2, 4, 6, 8, 10, 12, 14). (**C**) Gene expression of clone84, clone177, clone261.1, clone261.2, clone310 and clone460 in cultures of Caco-2 cells was analyzed by RT-PCR. A linear range of amplification was shown. (**D**) Analysis of HNF4a *in vitro* binding to further putative binding sites in promoters of CHIP clones. Electrophoretic mobility shift assays with 2,5 µg Caco-2 cell nuclear extract and oligonucleotides (GS65, lane 1 and 2; GS76, lane 3 and 4; GS82, lane 5 and 6; GS70, lane 7 and 8) corresponding to putative HNF4α binding-sites within the promoters of CHIP clones as ³²P labeled probe. In supershift assays an antibody directed against HNF4α (+) was added (lane 2, 4, 6, 8).

### Figure 8 Biological functions of HNF4α gene targets.

76 well-known HNF4α gene targets are assigned to functions in percent. The 13 novel HNF4α gene targets described in this study are assigned to functions with their gene names.

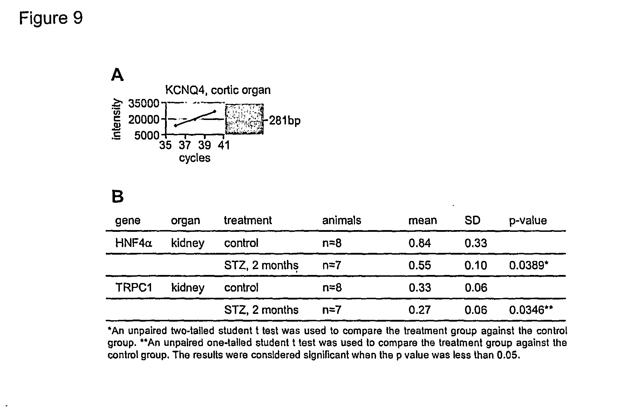

### Figure 9 Supplemental

A. Gene expression of KCNQ4 in rat cortic organs (sensory hair cells, supporting cells, auditory nerve fibres) was analyzed by RT-PCR. A linear range of amplification cycles was shown. B. HNF4α target regulation in STZ-induced diabetic rats. HNF4α and TRPC1 gene expression was measured with real-time RT-PCR. Gene expression levels were normalized to mitATPase.

## Claims

1. Use of one or more genes with an HNF4α binding site to screen for and to identify drugs against tumor growth, wherein the one or more genes are selected from the group of C20orf13, KJAA0774, EPS15R, PLCB1, UGTREL1.

2. A method of identifying compounds directed against tumor growth, wherein one or more genes with an HNF4α binding site are incubated with a compound to be tested and changes in the expression or transcriptional activity of said genes are determined, and wherein the one or more genes are selected from the group of C20orf13, KIAA0774, EPS15R, PLCB1, UGTREL1.

3. Use of an HNF4α chromatin immunoprecipitation assay (ChIP-assay) to screen for and to identify drugs against tumor growth, **characterized by** the steps formaldehyde crosslink cells,
isolate and sonicate nuclei,
first immunoprecipitation,
second immunoprecipitation,
reverse crosslink / DNA purification,
protein digest / DNA purification,
create blunt end fragments,
clone unknown target sequences,
sequencing,
annotation, and
in vivo conformation,
wherein one or more target genes of HNF4α selected from the group of C20ort13, KIAA0774, EPS15R, PLCB1, UGTREL1 are identified.

4. Use according to claim 3, wherein an antibody directed against HNF4α is used.

## Patentansprüche

1. Verwendung eines oder mehrerer Gene mit einer HNF4α Bindungsstelle zum Durchmustern und Identifizieren von Arzneimitteln gegen Tumorwachstum, wobei das oder die Gene ausgewählt sind aus der Gruppe C20orf13, KIAA0774, EPS15R, PLCB1, UGTREL1.

2. Verfahren zur Identifizierung gegen Tumorwachstum einsetzbarer Verbindungen,
wobei eines oder mehrere Gene mit einer HNF4α Bindungsstelle zusammen mit einer zu testenden Verbindung inkubiert werden und Veränderungen in der Expression oder der Transkriptionsaktivität besagter Gene bestimmt werden, und
wobei das oder die Gene ausgewählt sind aus der Gruppe C20orf13, KlAA0774, EPS15R, PLCB1, UGTREL1.

3. Verwendung einer HNF4α Chromatin-Immunopräzipitation (ChIP-Assay) zum Durchmustern und Identifizieren von Arzneimitteln gegen Tumorwachstum, **gekennzeichnet durch** die Schritte:
Formaldehyd-Vernetzung der Zellen
Isolierung der Zellkerne und Behandlung mit Schallenergie
Erste Immunopräzipitation
Zweite Immunopräzipitation,
Reversion der Vernetzung / DNA Aufreinigung
Proteinverdau / DNA Aufreinigung
Erzeugung von Blunt-End Fragmenten
Klonen unbekannter Zielsequenzen
Sequenzierung
Annotation und
Bestätigung *in vivo,*
wobei ein oder mehrere Zielgene von HNF4α, ausgewählt aus der Gruppe C20orf13, KIAA0774, EPS15R, PLCB1, UGTREL1, identifiziert werden.

4. Verwendung gemäß Anspruch 3, wobei ein gegen HNF4α gerichteter Antikörper eingesetzt wird.

## Revendications

1. Utilisation d'un ou de plusieurs gènes comprenant un site de liaison pour HNF4α pour cribler et identifier les drogues contre la croissance de tumeurs, dans laquelle le ou les gènes sont sélectionnés dans le groupe de C20orf13, KlAA0774, EPS15R, PLCB1, UGTREL1.

2. Une méthode d'identification des composés dirigés contre la croissance de tumeurs, dans laquelle un ou plusieurs gènes avec un site de liaison pour HNF4α sont incubés avec un composé à tester et les changements dans l'expression ou l'activité transcriptionnelle des dits gènes sont déterminés, et dans lequel un ou plusieurs gènes sont sélectionnés dans le groupe de C20orf13, KIAA0774, EPS15R, PLCB1, UGTREL1.

3. Utilisation d'une analyse par immunoprécipitation de chromatine pour HNF4α (analyse ChIP) pour cribler et identifier les drogues contre la croissance de tumeurs, **caractérisée par** les étapes
crosslink par formaldéhyde de cellules,
isolation et sonification les noyaux,
première immunoprécipitation,
deuxième immunoprécipitation,
reverse crosslink / purification de l'ADN,
digestion de protéines / purification de l'ADN,
création de fragments à extrémités franches,
clonage de séquences cibles inconnues,
séquençage,
annotation et
conformation in vivo,
dans lesquels un ou plusieurs gènes cibles HNF4α sélectionnés dans le groupe de C20orf13, KIAA0774, EPS15R, PLCB1, UGTREL1 sont identifiés.

4. Utilisation conformément à la revendication 3, dans laquelle un anticorps dirigé contre HNF4α est utilisé.
